# FASCICULE DE BREVET EUROPEEN

(11) **EP 0 374 054 B1**
(45) Date de publication et mention de la délivrance du brevet: **06.10.1993**
(21) Numéro de dépôt: 89403506.2
(22) Date de dépôt: 15.12.1989
(51) Int. Cl.: C07D 295/135, A61K 31/40, C07D 207/20, C07D 307/52, A61K 31/34, C07D 241/04, A61K 31/50

(54) **Composés dérivés de l'indane, leur procédé et les intermédiaires de préparation, leur application comme médicaments et les compositions pharmaceutiques les renfermant**
Indanderivate, Verfahren zu ihrer Herstellung und erhaltene Zwischenprodukte, ihre Verwendung als Arzneimittel und diese enthaltende pharmazeutische Zusammensetzungen
Indane derivatives, process for their preparation and intermediates obtained, their use as medicines, and pharmaceutical compositions containing them

(30) Priorité: 16.12.1988 FR 8816605
(43) Date de publication de la demande: 20.06.1990
(73) Titulaire: ROUSSEL-UCLAF, 75007 Paris (FR)
(72) Inventeur: Clemence, François, F-75009 Paris (FR); Fortin, Michel, F-75018 Paris (FR); Hamon, Gilles, F-93340 Le Raincy (FR); Le Martret, Odile, F-75016 Paris (FR); Moura, Anne-Marie, F-75014 Paris (FR)
(74) Mandataire: Bourgouin, André

(56) Documents cités:
- EP-A- 0 005 821
- EP-A- 0 258 096
- EP-A- 0 260 555
- EP-A- 0 272 982
- GB-A- 2 096 607
- CHEMICAL ABSTRACTS, vol. 108, no. 13, 28 mars 1988, page 577, résumé no. 111935b, Columbus, Ohio, US; M.E. SHABAN et al.: "Behavior of 2-bromo-1-indanol towards some amines"

## Description

Dans la demande de brevet européen n° 87401734.6 déposée le 24 juillet 1987 et publiée sous le n° 0 258 096 la demanderesse a décrit des composés dérivés de l'indane de formule (I): 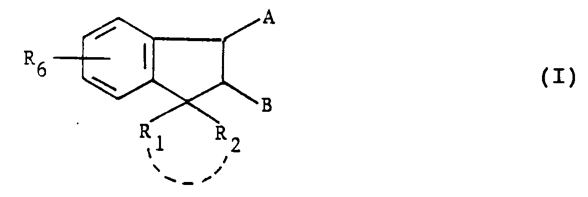
dans laquelle R₆ représente un atome d'hydrogène, un atome d'halogène, un radical alkyle ou alcoxy renfermant de 1 à 5 atomes de carbone et dans laquelle R₁ et R₂, identiques ou différents, représentent un atome d'hydrogène ou un radical alkyle renfermant de 1 à 5 atomes de carbone ou R₁ et R₂ forment ensemble avec l'atome de carbone auquel ils sont liés un cycle cycloalkyle renfermant entre 3 et 6 atomes de carbone et pouvant contenir éventuellement un hétéroatome tel qu'un atome d'oxygène ou de soufre ou d'azote et dans laquelle A et B sont tels que l'un des substituants A ou B est choisi dans :
- le groupe de formule : 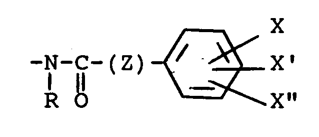 R étant un atome d'hydrogène ou un radical alkyle renfermant de 1 à 5 atomes de carbone, Z une chaîne alkylène linéaire -(CH₂)ₙ-, n étant un nombre entier pouvant varier entre 0 et 5 ou une chaîne alkylène ramifiée renfermant de 2 à 8 atomes de carbone ou un groupement -(CH₂)-O-, X, X' et X'', identiques ou différents, représentent un atome d'hydrogène, un radical alkyle ou alcoxy renfermant de 1 à 4 atomes de carbone, un atome d'halogène, un radical hydroxyle, trifluorométhyle, nitro, amino, monoalkyle ou dialkylamino ou sulfoamino et l'autre des substituants A ou B est choisi dans :
- le groupe de formule : 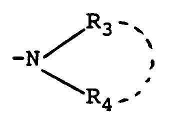 R₃ et R₄, identiques ou différents, représentent un atome d'hydrogène ou un radical alkyle renfermant de 1 à 5 atomes de carbone ou R₃ et R₄ forment ensemble, avec l'atome d'azote auquel ils sont liés, un hétérocycle à 5 ou 6 chaînons comportant éventuellement un autre hétéroatome tel que l'oxygène, l'azote ou le soufre, l'atome d'azote étant éventuellement substitué par un radical alkyle renfermant de 1 à 4 atomes de carbone, lesdits composés de formule (I) pouvant être dans toutes les formes énantiomères et diastéréoisomères possibles et sous forme de sels d'addition avec les acides.

La demanderesse a également décrit un procédé de préparation de ces composés ainsi que leur application comme médicaments et les compositions pharmaceutiques les renfermant.

La demande de brevet européen citée ci-dessus a décrit notamment, à l'exemple 9, le [trans (±)] N-[2,3-dihydro 2-(1-pyrrolidinyl) 1H-indèn-1-yl] N-méthyl 3-nitro benzèneacétamide et son chlorhydrate de formule : 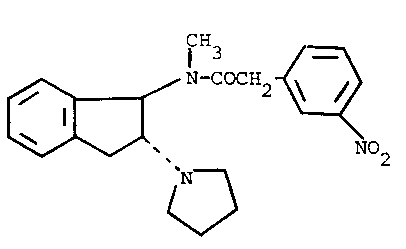
ce composé est obtenu à partir de la [trans (±)] 2,3-dihydro N-méthyl 2-(1-pyrrolidinyl) 1H-indèn-1-amine décrite à l'exemple 1 stade A de la demande de brevet citée ci-dessus. Enfin, la séparation des isomères de la [trans (±)] 2,3-dihydro N-méthyl 2-(1-pyrrolidinyl) 1H-indèn 1-amine en ses isomères A et B est décrite à l'exemple 16 stade A de la demande de brevet européen désignée ci-dessus.

Dans le brevet européen N° 0.260.555 sont décrits notamment des dérivés benzocycloalkanes trans 1,2-diamines présentant des propriétés analgésiques et/ou diurétiques.

La présente demande concerne de nouveaux dérivés de l'indane, leur procédé de préparation, les nouveaux intermédiaires ainsi obtenus, leur application à titre de médicaments et les compositions pharmaceutiques les renfermant.

L'invention a pour objet les composés de formule (I) : 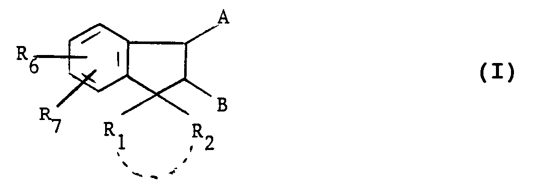
dans laquelle R₁ et R₂, identiques ou différents, représentent un atome d'hydrogène ou un radical alkyle renfermant de 1 à 5 atomes de carbone ou R₁ et R₂ forment ensemble avec l'atome de carbone auquel ils sont liés un cycle cycloalkyle renfermant entre 3 et 6 atomes de carbones et pouvant contenir éventuellement un hétéroatome tel qu'un atome d'oxygène ou de soufre ou d'azote et ou bien R₆ et R₇, identiques ou différents, représentent un atome d'halogène, un radical alkyle ou alcoxy renfermant de 1 à 5 atomes de carbone et A et B sont tels que l'un des substituants A ou B est choisi dans
- le groupe de formule 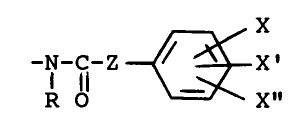 R étant un atome d'hydrogène ou un radical alkyle renfermant de 1 à 5 atomes de carbone, Z une chaîne alkylène linéaire - (CH₂)ₙ-, n étant un nombre entier pouvant varier entre 0 et 5 ou une chaîne alkylène ramifiée renfermant de 2 à 8 atomes de carbone ou un groupement -CH₂-O-, X, X' et X'', identiques ou différents, représentent un atome d'hydrogène, un radical alkyle ou alkoxy renfermant de 1 à 4 atomes de carbone, un atome d'halogène, un radical hydroxyle, trifluorométhyle, nitro, amino, monoalkyle ou dialkylamino ou sulfoamino et l'autre des substituants A ou B est choisi dans
- le groupe de formule 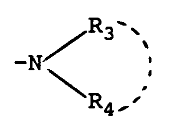 R₃ et R₄, identiques ou différents, représentent un atome d'hydrogène ou un radical alkyle renfermant de 1 à 5 atomes de carbone ou R₃ et R₄ forment ensemble avec l'atome d'azote auquel ils sont liés un hétérocycle à 5 ou 6 chaînons comportant éventuellement un autre hétéroatome tel que l'oxygène, l'azote ou le soufre, l'atome d'azote étant éventuellement substitué par un radical alkyle renfermant de 1 à 4 atomes de carbone,
   ou bien R₆ et R₇ représentent un atome d'hydrogène et
- soit B représente un radical pyrrolidine et A représente un groupe 3,5-bis (1,1-diméthyléthyl) 4-hydroxy N-méthyl benzamide, N-méthyl 3-nitrobenzène acétamide ou 3,4-dichloro N-méthyl benzène acétamide, ces deux derniers produits étant nécessairement sous forme d'énantiomères purs,
- soit A représente un radical 3,4-dichloro N-méthyl benzène acétamide et B représente un groupe méthyl (2-phényléthyl) amino ou (2-furylméthyl) méthylamino ou 4-(2-méthoxyphényl) 1-pipérazinyle,
   ou bien R₆ et R₇ sont tels que l'un représente un atome d'hydrogène et l'autre un atome de chlore, B représente un radical pyrrolidine et A représente un groupe N-méthyl 3-nitro benzène acétamide,
   lesdits composés de formule (I) pouvant être sous toutes leurs formes stéréoisomères possibles ainsi que leurs hydrates et les sels d'addition avec les acides de ces composés et de leurs hydrates, ainsi que le trans (±) 3,4-dichloro N-[2,3-dihydro 2-(2,5-dihydro 1H-pyrrol-1-yl) 1H-indèn-1-yl] N-méthyl benzène acétamide sous forme de base libre ou de chlorhydrate.

Les sels d'addition avec les acides minéraux ou organiques peuvent être, par exemple, les sels formés avec les acides chlorhydrique, bromhydrique, nitrique, sulfurique, phosphorique, acétique, propionique, formique, benzoïque, maléique, fumarique, succinique, tartrique, citrique, oxalique, glyoxylique, aspartique, alcanesulfoniques tels que l'acide méthanesulfonique et arylsulfoniques, tels que l'acide benzènesulfonique.

Les composés ci-dessus ainsi que leurs sels peuvent se présenter sous forme d'hydrates ou sous forme anhydre. On a aussi décrit ci-après dans les exemples des chlorhydrates hydratés et des chlorhydrates anhydres.

L'invention a notamment pour objet les composés de formule (I), caractérisés en ce que les groupements A et B sont en confifuration trans ainsi que leurs hydrates et leurs sels d'addition avec les acides et ceux caractérisés en ce que dans le groupe 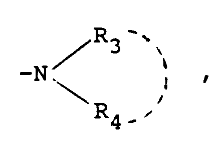
R₃ et R₄ forment avec l'atome d'azote auquel ils sont liés l'hétérocycle pyrrolidine, ainsi que leurs hydrates et leurs sels d'addition avec les acides.

L'invention a aussi particulièrement pour objet les composés de formule (I), caractérisés en ce que le groupe 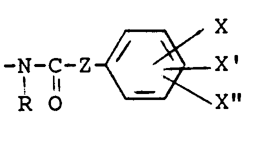
représente 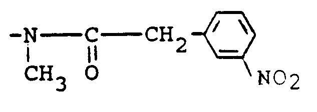
ainsi que leurs hydrates et leurs sels d'addition avec les acides et ceux caractérisés en ce que R₁ et R₂ représentent tous deux un atome d'hydrogène, ainsi que leurs hydrates et leurs sels d'addition avec les acides.

L'invention concerne aussi particulièrement les composés de formule (I), caractérisés en ce que R₆ et R₇, identiques ou différents représentent un atome d'hydrogène ou de chlore, ainsi que leurs hydrates et leurs sels d'addition avec les acides et tout particulièrement :
- le (1S,2S) N-[2,3-dihydro 2-(1-pyrrolidinyl) 1H-indèn-1-yl] N-méthyl 3-nitro benzène acétamide et son chlorhydrate anhydre,
- le trans (±) N-[5,6-dichloro 2,3-dihydro 2-(1-pyrrolidinyl) 1H-indèn-1-yl] N-méthyl 3-nitro benzène acétamide et son chlorhydrate,
- le trans (±) N-[5-chloro 2,3-dihydro 2-(1-pyrrolidinyl) 1H-indèn-1-yl] N-méthyl 3-nitro benzène acétamide et son chlorhydrate,
L'invention a aussi pour objet un procédé de préparation des produits de formule (I) dont les groupements A et B sont en configuration trans, caractérisé en ce que l'on condense le produit de formule (II) : 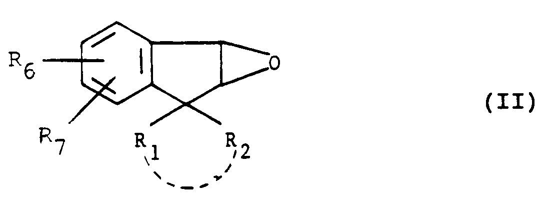
R₆, R₇, R₁ et R₂ ayant toutes les significations données précédemment,
- soit avec une amine de formule (III) : 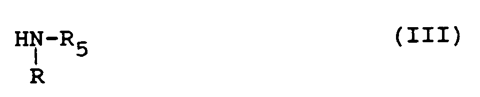 R ayant toutes les significations données précédemment et R₅ étant un groupement protecteur de la fonction amine et notamment un groupement benzyle, pour obtenir un produit de formule (IV) : 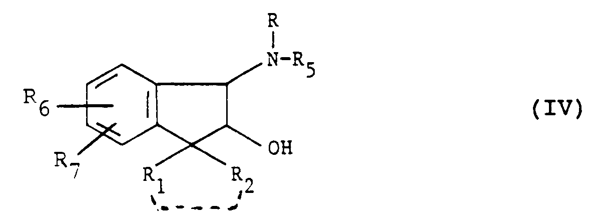 dont on active la fonction hydroxyle et que l'on condense avec une amine de formule (V) : 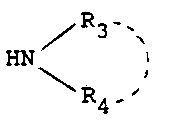 dans laquelle R₃ et R₄ ont les significations données précédemment pour obtenir un produit de formule (VI) : 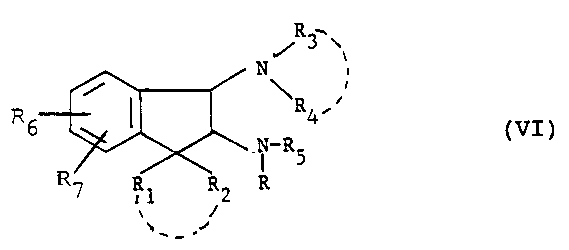 dont on élimine le groupement protecteur R₅ de la fonction amine pour obtenir un produit de formule (VII) : 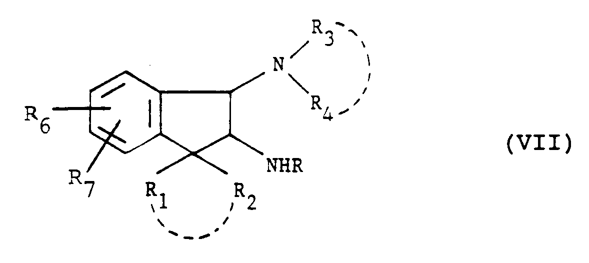 que l'on traite avec un acide de formule (VIII) ou un dérivé fonctionnel de cet acide : 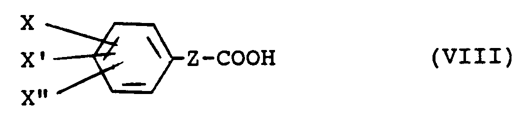 dans laquelle Z, X, X' et X'' ont toutes les significations précédentes pour obtenir un produit de formule (I) dans laquelle A représente le groupe 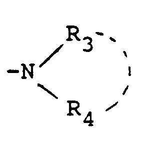 et B le groupe 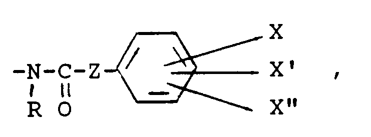
- soit avec une amine de formule : 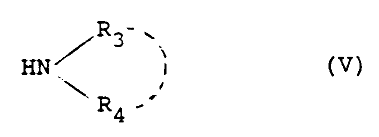 pour obtenir un produit de formule (IX) : 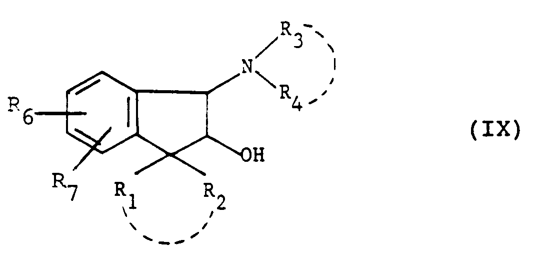 dont on active la fonction hydroxyle et que l'on traite avec une amine de formule (X) :

   NH₂R (X)

   dans laquelle R a la signification précédente pour obtenir un produit de formule (XI) : 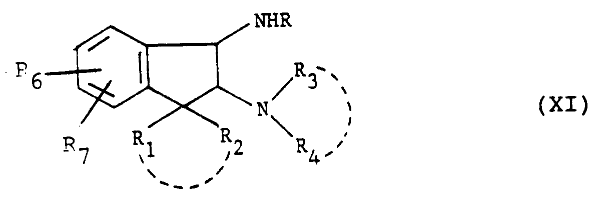 que l'on condense avec un acide de formule (VIII) ou un dérivé fonctionnel de cet acide : 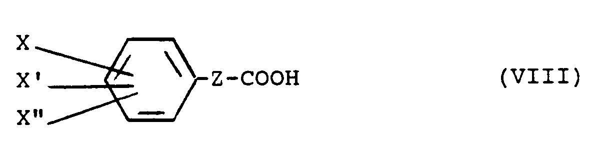 dans laquelle Z, X, X' et X'' ont les significations précédentes pour obtenir un produit de formule (I) dans laquelle A représente le groupe 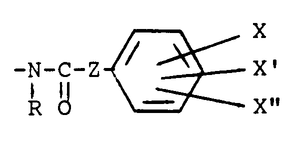 et B le groupe 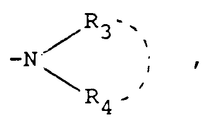 lesdits composés de formule (I) pouvant être dédoublés pour obtenir les formes optiquement actives et que l'on traite, si désiré, avec un acide minéral ou organique, pour obtenir un sel.

Dans les conditions préférentielles du procédé de l'invention :
- Le chlorure de méthanesulfonyle est utilisé pour activer la fonction hydroxyle des produits de formule (IV) et des produits de formule (IX).
- Dans les produits de formule (VI), le groupement de protection R₅ est un radical benzyle qui peut être éliminé par hydrogénation catalytique. Le catalyseur que l'on utilise est de préférence le palladium.
- L'activation de la fonction hydroxyle des composés de formule (VIII) pour réaliser la condensation avec le composé de formule (VII) ou (XI) s'effectue en présence de carbonyldiimidazole. On peut également activer les acides de formule (VIII) sous la forme d'un chlorure d'acide ou d'anhydride mixte.
- Les produits de formule (I) peuvent être dédoublés par les méthodes usuelles.

Les produits de formule (I) pour lesquels les groupements A et B sont en configuration cis peuvent être préparés notamment selon le schéma suivant : 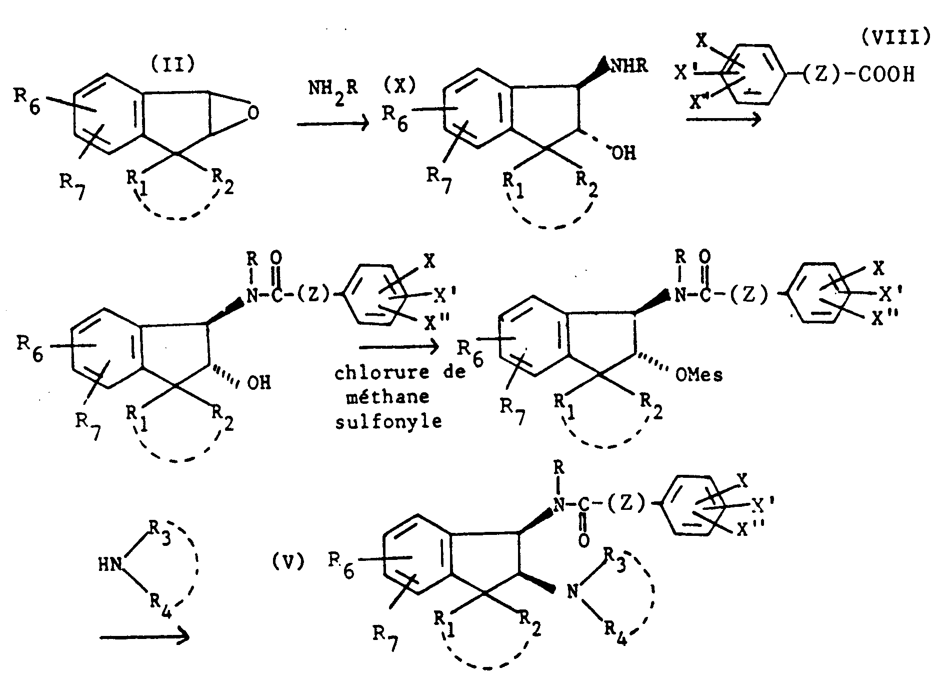
Les composés de la présente demande et leurs formes hydratées et/ou salifiées présentent d'intéressantes propriétés pharmacologiques. Ils présentent en particulier une forte affinité pour les récepteurs opiacés et notamment pour les récepteurs K et sont doués de propriétés analgésiques centrales.

Les composés de la présente invention et leurs formes hydratées et/ou salifiées présentent en outre une activité anti-arythmique et une activité diurétique.

Ils peuvent également être actifs dans le traitement de l'ischémie cérébrale.

Ces propriétés justifient leur application en thérapeutique et l'invention a également pour objet, à titre de médicaments, les composés de la présente demande, ainsi que leurs hydrates et leurs sels d'addition avec les acides pharmaceutiquement acceptables.

Parmi les médicaments objet de l'invention, on retient de préférence les médicaments caractérisés en ce qu'ils sont constitués par les nouveaux dérivés de l'indane répondant à la formule (I) dans lesquels A et B sont de configuration trans, ceux dans lesquels R₃ et R₄ forment avec l'atome d'azote auquel ils sont liés un hétérocycle pyrrolidine, ceux dans lesquels le groupe 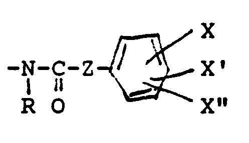
représente 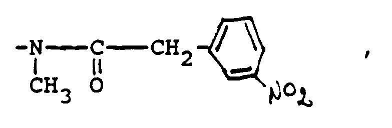
ceux dans lesquels R₁ et R₂ représentent tous deux un atome d'hydrogène, ceux dans lesquels R₆ et R₇ identiques représentent tous deux un atome d'hydrogène ou tous deux un atome de chlore, ainsi que leurs hydrates et leurs sels d'addition avec les acides.

La présente invention a tout particulièrement pour objet, à titre de médicament :
- le (1S,2S) N-[2,3-dihydro 2-(1-pyrrolidinyl) 1H-indèn-1-yl] N-méthyl 3-nitro benzèneacétamide et son chlorhydrate anhydre,
- le trans (±) N-[5,6-dichloro 2,3-dihydro 2-(1-pyrrolidinyl) 1H-indèn-1-yl] N-méthyl 3-nitro benzène acétamide et son chlorhydrate,
- le trans (±) N-[5-chloro 2,3-dihydro 2-(1-pyrrolidinyl) 1H-indèn-1-yl] N-méthyl 3-nitro benzène acétamide et son chlorhydrate.

Les médicaments, objets de l'invention, permettent notamment de soulager une douleur quelle qu'en soit l'origine, par exemple une douleur de nature musculaire, articulaire ou nerveuse.

Ils peuvent être aussi utilisés dans le traitement des douleurs dentaires, des migraines, du zona dans le traitement des douleurs intenses, en particulier rebelles aux antalgiques périphériques, par exemple au cours du processus néoplasique, dans le traitement des pancréatites, coliques néphrétiques ou biliaires, dans le traitement des douleurs post-opératoires et post-traumatiques.

La posologie varie notamment en fonction de la voie d'administration, de l'affection traitée et du sujet en cause.

Par exemple, chez l'adulte, elle peut varier entre 20 et 400 mg de principe actif par jour, par voie orale et entre 5 et 100 mg par jour, par voie parentérale.

Les médicaments objets de l'invention, trouvent aussi leur emploi dans le traitement des arythmies.

La dose usuelle, variable selon le dérivé utilisé, le sujet et l'affection en cause peut être par exemple de 50 mg à 1 g par jour.

Par voie orale, le principe actif peut être administré à la dose quotidienne de 200 mg à 800 mg, par exemple pour le traitement des arythmies ventriculaires, supraventriculaires et jonctionnelles, soit environ de 3 mg à 12 mg par kilogramme de poids corporel.

Les médicaments, objets de l'invention, peuvent aussi être utilisés dans le traitement des syndromes oedémateux, de l'insuffisance cardiaque, de certaines obésités, des cirrhoses, dans le traitement des oedèmes sévères et réfractaires, en particulier ceux de l'insuffisance cardiaque congestive et dans le traitement au long cours de l'hypertension artérielle.

La dose quotidienne de principe actif est variable. Elle peut être par exemple de 60 à 100 mg/jour par voie orale.

L'invention s'étend aux compositions pharmaceutiques renfermant comme principe actif les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, les dispersants ou émulsifiants, les conservateurs.

Les produits de départ de formule (II) pour lesquels R₁ et R₂ sont des atomes d'hydrogène ou des radicaux alcoyles renfermant de 1 à 5 atomes de carbone sont préparés par oxydation de l'indène correspondant.

Les autres produits de formule (II), et notamment ceux pour lesquels R₁ et R₂ forment un tétrahydropyranne, sont préparés selon le schéma suivant : 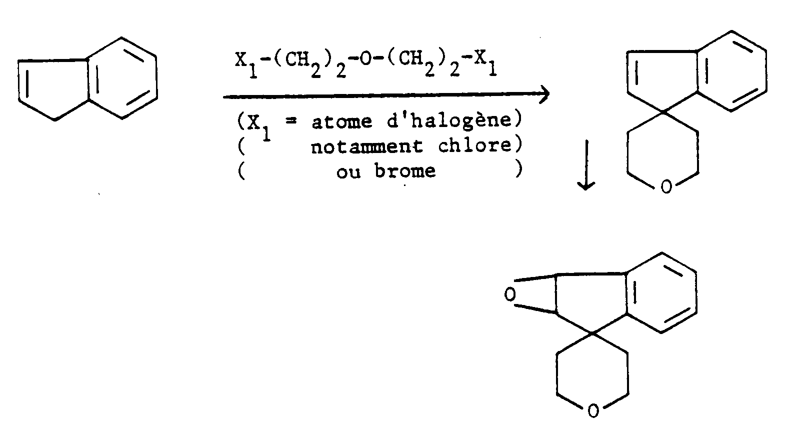
L'invention concerne aussi, à titre de produits industriels nouveaux, les produits de formules (IV), (VI), (VII), (IX), (XI) dans laquelle R₆ et R₇ ne représentent pas tous deux un atome d'hydrogène.

Les exemples donnés ci-après illustrent l'invention sans toutefois la limiter.

### EXEMPLE 1 : (1S,2S) N-[2,3-dihydro 2-(1-pyrrolidinyl) 1H-indèn-1-yl] N-méthyl 3-nitro benzèneacétamide et son chlorhydrate hydraté ou anhydre.

### a) Base libre.

On agite une heure à température ambiante un mélange de 381 mg d'acide 3-nitrophényl acétique, 341 mg de carbonyldiimidazole et 3 cm³ de tétrahydrofuranne et ajoute en une seule fois 350 mg de trans 2,3-dihydro N-méthyl 2-(1-pyrrolidinyl) 1H-indèn-1-amine isomère B (alpha_{D} = +10,5° c = 1 % dans CH₃OH) telle que décrite dans la demande de brevet européen EP 0.258.096 exemple 16A. On rince avec 3 cm³ de tétrahydrofuranne et agite la suspension obtenue pendant 30 heures à température ambiante. On distille à sec le tétrahydrofuranne sous pression réduite, reprend par 30 cm³ d'acétate d'éthyle et lave par 10 cm³ d'une solution aqueuse saturée de bicarbonate de sodium puis à l'eau. On réextrait les lavages avec de l'acétate d'éthyle, sèche, filtre, rince et distille à sec. On obtient 594 mg de produit attendu. On chromatographie 564 mg sur silice (éluant : acétate d'éthyle à 2 % de triéthylamine et récupère 502 mg de produit purifié.

### b) Chlorhydrate hydraté.

On dissout 470 mg de produit purifié dans 3 cm³ d'acétate d'éthyle saturé d'eau, filtre, rince à l'acétate d'éthyle saturé d'eau (7 cm³ au total) et ajoute 0,3 cm³ de solution éthanolique d'acide chlorhydrique 6,6 N. On concrète la gomme formée, essore les cristaux blancs obtenus et rince à l'acétate d'éthyle puis à l'éther diéthylique. On sèche et obtient 447 mg de produit sous forme de chlorhydrate hydraté.
F = 140-142°C ; alpha_{D} = +74° ± 1,5° (c = 0,9 % H₂O.)
Dichroïsme circulaire dans EtOH.
Max 206 nm delta epsilon = +19
Max 216 nm delta epsilon = +17,4
Max 221 nm delta epsilon = +15,5
Inflexion 320 nm delta epsilon = +0,05
Max 265 nm delta epsilon = +0,93
Max 272 nm delta epsilon = +0,98.

### c) Chlorhydrate non solvaté.

En partant de 3,24 g du même produit trans 2,3-dihydro N-méthyl 2-(1-pyrrolidinyl) 1H-indèn-1-amine isomère B et en opérant selon le mode opératoire décrit ci-dessus, on isole 6,9 g de produit attendu. On dissout ce produit à 20°C dans 20 cm³ de diméthoxy éthane à 2 % d'eau et ajoute en laissant chauffer 3,5 cm³ de solution éthanolique d'acide chlorhydrique. On amorce la cristallisation à chaud, glace 30 minutes à 0, +5°C, essore à 0°C, rince l'aide de diméthoxyéthane puis à l'éther diéthylique. On sèche à 80°C sous pression réduite et obtient 5,83 g de chlorhydrate F 220°C. On dissout ce produit dans 305 cm³ de diméthoxyéthane à 5 % d'eau, filtre, rince et concentre sous pression réduite jusqu'à un volume de 70 cm³. On isole ensuite de la même manière 5,66 g de chlorhydrate non solvaté F = 220-222°C (alpha_{D} = +77° ± 2°, c = 1 % dans H₂O).

### EXEMPLE 2 : (1R,2R) N-[2,3-dihydro 2-(1-pyrrolidinyl) 1H-indèn-1-yl] N-méthyl 3-nitro benzèneacétamide et son chlorhydrate sesqui hydraté ou anhydre.

### a) Base libre.

En opérant comme à l'exemple 1 a) ci-dessus au départ de 1,045 g de trans 2,3-dihydro N-méthyl 2-(1-pyrrolidinyl) 1H-indèn-1-amine isomère A (alpha_{D} = -10,5° c = 1 % dans méthanol) décrit à l'exemple 16A de la demande de brevet européen EP 0.258.096, on obtient 1,77 g de produit purifié.

### b) Chlorhydrate sesquihydraté.

A partir de 1,688 g de produit obtenu en a) et en opérant comme à l'exemple 1 b), on obtient 1,699 g de chlorhydrate sesquihydraté.
Alpha_{D} = -71,5° ± 1,5° c = 1 % H₂O.
Dichroïsme circulaire dans EtOH.
Max 210 nm delta epsilon = -16,9
Max 215 nm delta epsilon = -17
Max 221 nm delta epsilon = -15,6
Inflexion 323 nm delta epsilon = -0,08
Max 266 nm delta epsilon = -1
Max 272 nm delta epsilon = -1.

### c) Chlorhydrate anhydre.

En opérant comme à l'exemple 1 c) au départ de 0,43 g de l'isomère A décrit à l'exemple 16A de la demande européenne EP 0.258.096, on obtient 661 mg de chlorhydrate anhydre. On dissout celui-ci dans 27 cm³ de diméthoxyéthane à 5 % d'eau et obtient après concentration à volume réduit : 432 mg de chlorhydrate anhydre. F = 220-222°C.
Alpha_{D} = -76° ± 1,5° c = 1 % dans H₂O.

### EXEMPLE 3 : trans (±) 3,5-bis (1,1-diméthyléthyl) N-[2,3-dihydro 2-(1-pyrrolidinyl) 1H-indèn-1-yl] 4-hydroxy N-méthyl benzamide et son chlorhydrate.

On agite 1 heure à température ambiante sous atmosphère inerte 7 g d'acide 3,5-diterbutyl 4-hydroxy benzoïque et 7 cm³ de chlorure de thionyle. On agite ensuite 15 minutes à 60°C, élimine le solvant sous pression réduite, empâte le résidu cristallisé au pentane, essore et sèche. On obtient 7 g de chlorure d'acide. On refroidit à 0°C 3 g de trans (±) 2,3-dihydro N-méthyl 2-(1-pyrrolidinyl) 1H-indèn-1-amine dans 30 cm³ de chlorure de méthylène et 1,93 cm³ de triéthylamine et ajoute lentement 4,2 g de chlorure d'acide préparé ci-dessus. On laisse revenir à température ambiante et agite pendant 4 heures. On élimine les solvants sous pression réduite, dilue à l'eau, extrait au chlorure de méthylène, lave à l'eau, sèche et élimine les solvants. On récupère 6 g de produit brut que l'on purifie par chromatographie sur silice (éluant : acétate d'éthyle-triéthylamine 9-1). On obtient 4 g de produit attendu sous forme de base. On dissout cette base dans 40 cm³ d'acétate d'éthyle, ajoute une solution saturée d'acide chlorhydrique dans l'éthanol, glace, essore et sèche à 80°C sous pression réduite. Après purification dans l'isopropanol, on obtient 3,4 g du chlorhydrate attendu. F > 260°C.

### EXEMPLE 4 : trans (±) N-[5-chloro 2,3-dihydro 2-(1-pyrrolidinyl) 1H-indèn-1-yl] N-méthyl 3-nitro benzène acétamide et son chlorhydrate.

On agite 1 heure à température ambiante 912 mg d'acide méta-nitrophényl acétique et 813 mg de carbonyldiimidazole dans 10 cm³ de tétrahydrofuranne, ajoute 1,7 cm³ de triéthylamine puis 1,55 g d'oxalate de trans (±) 5-chloro 2,3-dihydro N-méthyl 2-(1-pyrrolidinyl) 1H-indèn 1-amine et maintient 6 heures sous agitation. On élimine le solvant sous pression réduite, reprend le résidu dans 30 cm³ d'acétate d'éthyle, lave la phase organique avec une solution aqueuse à 10% de bicarbonate de sodium, sèche, concentre à sec et recueille 1,73 g de produit attendu sous forme de base. On dissout 1,45 g de cette base dans 10 cm³ d'isopropanol à 20°C, ajoute 1 cm³ de solution éthanolique de chlorure d'hydrogène et concentre partiellement sous pression réduite. On triture le produit cristallisé dans 10 cm³ d'isopropanol, essore, rince à l'isopropanol puis à l'éther, sèche à 60°C et obtient 1,19 g de produit attendu, que l'on recristallise dans l'éthanol. F = 223-225°C.

| Analyse : | | | | | | | | |
|---|---|---|---|---|---|---|---|---|
| Calculés | C% | 58,67 | H% | 5,59 | N% | 9,33 | Cl% | 15,74 |
| Trouvés | | 58,6 | | 5,6 | | 9,3 | | 15,7 |

### EXEMPLE 5 : 1S,trans N-[5-chloro 2,3-dihydro 2-(1-pyrrolidinyl) 1H-indèn-1-yl] N-méthyl 3-nitro benzène acétamide et son méthanesulfonate.

### A) Trans (-) N-méthyl 5-chloro 2,3-dihydro 2-(1-pyrrolidinyl) 1H-indèn 1-amine (isomère A).

On ajoute à 20°C 50 cm³ d'éther éthylique dans 6,83 g d'oxalate de trans (±) N-méthyl 5-chloro 2,3-dihydro 2-(1-pyrrolidinyl) 1H-indèn 1-amine (préparé comme à l'exemple 31 de la demande de brevet européen publiée sous le n° 0 258 096) en solution dans 10 cm³ d'eau. On ajoute lentement 10 cm³ de lessive de potasse, agite, décante, extrait à l'éther éthylique, sèche, concentre à sec sous pression réduite et recueille 4,25 g de base. On dissout 4,19 g de cette base dans 20 cm³ de méthanol et ajoute une solution comprenant 6,62 g d'acide L(-) di-p-tolyl tartrique dans 60 cm³ de méthanol. On amorce la cristallisation, maintient 16 heures à 20°C, glace 1 heure, essore les cristaux formés, les rince au méthanol puis à l'éther éthylique et obtient après recristallisation dans le méthanol 2,8 g de sel d'isomère A. F ≃ 180°C.
[alpha]_{D} = -33° ± 1,5° (c = 1% DMF).

On reprend 2,75 g du sel dans 10 cm³ d'eau et 50 cm³ d'éther et ajoute 3 cm³ de lessive de potasse. On élimine le précipité, extrait à l'éther, sèche et concentre à sec sous pression réduite. On recueille 1,077 g de produit attendu. [alpha]_{D} = -9° ± 2° (c = 0,5% méthanol).

### B) 1S,trans N-[5-chloro 2,3-dihydro 2-(1-pyrrolidinyl) 1H-indèn-1-yl] N-méthyl 3-nitro benzène acétamide et son méthanesulfonate.

On opère comme à l'exemple 1a) en utilisant 904 mg d'acide m-nitrophényl acétique, 809 mg de carbonyldiimidazole dans 10 cm³ de tétrahydrofuranne et 964 mg de l'amine isomère A (-) préparée au stade précédent, dans 3 cm³ de tétrahydrofuranne. On obtient 1,59 g de produit brut que l'on dissout dans 10 cm³ d'éthanol, ajoute 0,3 cm³ d'acide méthanesulfonique, laisse cristalliser à 20°C, essore, rince à l'éthanol puis à l'éther, sèche les cristaux à 60°C sous pression réduite et recueille 1,30 g de méthanesulfonate attendu après recristallisation dans l'éthanol. F = 215-216°C.
[alpha]_{D} = +82° ± 2° (c = 1% H₂O)
Dichroïsme circulaire dans EtOH

| | |
|---|---|
| max 227 nm | delta epsilon = 19,5 |
| infl. 295 nm | delta epsilon = 0,2 |

### EXEMPLE 6 : 1R,trans N-[5-chloro 2,3-dihydro 2-(1-pyrrolidinyl) 1H-indèn-1-yl] N-méthyl 3-nitro benzène acétamide et son méthanesulfonate.

### Stade A : Trans (+) N-méthyl 5-chloro 2,3-dihydro 2-(1-pyrrolidinyl) 1H-indèn 1-amine (isomère B).

On concentre à sec les eaux mères méthanoliques obtenues après filtration du sel isomère A préparé au stade A de l'exemple 5. On reprend le résidu dans l'eau, l'éther et la lessive de potasse comme indiqué à l'exemple 5 et obtient 3,2 g de produit sous forme de base que l'on dissout dans 80 cm³ de méthanol et 4,75 g d'acide D(+) p-tolyl tartrique monohydraté. On amorce la cristallisation, abandonne 3 heures et demie à 20°C, essore, et obtient après recristallisation dans le méthanol 3,28 g de sel d'isomère B. F ≃ 180°C.
[alpha]_{D} = +32° ± 1,5° (c = 1% DMF)
On reprend 3,21 g de ce sel, opère comme indiqué à l'exemple 5 stade A et recueille 1,267 g de produit attendu. [alpha]_{D} = +8,5° ± 2° (c = 0,5% méthanol)

### Stade B : 1R, trans N-[5-chloro 2,3-dihydro 2-(1-pyrrolidinyl) 1H-indèn-1-yl] N-méthyl 3-nitro benzène acétamide et son méthanesulfonate.

On opère comme au stade B de l'exemple 5 à partir de 501 mg de l'amine isomère B préparée ci-dessus et obtient 859 mg de produit attendu sous forme de base puis 694 mg de méthanesulfonate pur attendu. F = 215-216°C.
[alpha]_{D} = -87,5° ± 2° (c = 1% H₂O)
Dichroïsme circulaire dans EtOH

| | |
|---|---|
| max 226 nm | delta epsilon = -20 |
| infl. 290 nm | delta epsilon = - 0,2 |

### EXEMPLE 7 : trans (±) 3,4-dichloro N-[2,3-dihydro 2-(2,5-dihydro 1H-pyrrol-1-yl) 1H-indèn-1-yl] N-méthyl benzène acétamide et son chlorhydrate.

### Stade A : trans (±) 2,3-dihydro 1-(2,5-dihydro 1H-pyrrol-1-yl) 1H-indèn-2-ol.

Dans 7,4 g de 1a,6a-dihydro 6H-indeno [1,2-b] oxirène en solution dans 5 cm³ d'eau, on ajoute à 20°C 10 g de 2,5-dihydro 1H-pyrrole (2-butène dioate) en solution dans 20 cm³ d'eau en présence de 6 g de carbonate de sodium. On chauffe à 70°C pendant 4 heures, laisse revenir à température ambiante et ajoute 15 cm³ de lessive de soude. On extrait à l'acétate d'éthyle, sèche et concentre à sec sous pression réduite. On obtient 8,62 g de produit brut que l'on chromatographie sur silice (éluant : acétate d'éthyle puis acétate d'éthyle-triéthylamine 98-2).

### Stade B : Dinitrate de trans (±) 2,3-dihydro 2-(2,5-dihydro 1H-pyrrol-1-yl) N-méthyl 1H-indèn-1-amine.

On mélange à 20°C 3,03 g de produit préparé au stade A dans 25 cm³ de tétrahydrofuranne et 2,7 cm³ de triéthylamine. On refroidit la solution à -20°C, ajoute en 5 minutes 1,4 cm³ de chlorure de méthanesulfonyle en solution dans 2 cm³ de tétrahydrofuranne. On agite 15 minutes, laisse revenir à 0°C, ajoute 20 cm³ d'une solution éthanolique de méthylamine à 33%, agite en laissant revenir à température ambiante, distille le tétrahydrofuranne sous pression réduite, reprend le résidu dans 30 cm³ d'acétate d'éthyle, ajoute 40 cm³ de lessive de soude, diluée au demi, agite, décante et réextrait à l'acétate d'éthyle, concentre à sec et récupère 3,27 g de produit attendu sous forme de base que l'on dissout dans 10 cm³ d'acétate d'éthyle, ajoute à +10°C, 1,4 cm³ d'acide nitrique fumant en solution dans 10 cm³ d'acétate d'éthyle, décante, reprend le résidu dans 10 cm³ d'éthanol, chauffe au reflux, amorce la cristallisation, essore les cristaux et les sèche à 20°C sous pression réduite et obtient 3,83 g de produit attendu que l'on recristallise dans l'éthanol. F = 172-173°C.

### Stade C : trans (±) 3,4-dichloro N-[2,3-dihydro 2-(2,5-dihydro 1H-pyrrol-1-yl) 1H-indèn-1-yl] N-méthyl benzène acétamide et son chlorhydrate.

On opère comme à l'exemple 4 en utilisant 533 mg d'acide 3,4-dichlorophényl acétique et 680 mg de dinitrate de trans (±) 2,3-dihydro 2-(2,5-dihydro 1H-pyrrol-1-yl) N-méthyl 1H-indèn-1-amine obtenu au stade B. On obtient 954 mg de produit attendu sous forme de base puis 530 mg de chlorhydrate. F = 237-239°C.

| Analyse : C₂₂H₂₂Cl₂N₂O, HCl | | | | | | | | |
|---|---|---|---|---|---|---|---|---|
| Calculé | C% | 60,36 | H% | 5,29 | N% | 6,40 | Cl% | 24,29 |
| Trouvé | | 60,3 | | 5,2 | | 6,3 | | 24,0 |

### EXEMPLE 8 : trans (±) N-[5,6-dichloro 2,3-dihydro 2-(1-pyrrolidinyl) 1H-indèn-1-yl] N-méthyl 3-nitro benzène acétamide et son chlorhydrate.

### Stade A : trans (±) 4,5-dichloro 1aH-indeno [1,2-b] oxirène.

On refroidit à -7°C 8 g de 5,6 dichloro 1H-indène dans 56 cm³ de chlorure de méthylène, 140 cm³ d'une solution aqueuse à 10% de bicarbonate de soude et 12 g de bicarbonate de soude et ajoute 15,6 g d'acide métachloroperbenzoïque en maintenant la température inférieure à 10°C. On agite ensuite 16 heures à température ambiante, ajoute 0,7 g d'hyposulfite de sodium, extrait au chlorure de méthylène, réunit les phases organiques, les lave à l'eau, sèche et élimine les solvants sous pression réduite et obtient 9,2 g de produit attendu que l'on utilise tel quel dans le stade suivant.

### Stade B : trans (±) 5,6-dichloro 2,3-dihydro 1-(1-pyrrolidinyl) 1H-indèn-2-ol.

On ajoute en 20 minutes le mélange de 10 cm³ de pyrrolidine et 10 cm³ d'eau à 12 g du produit obtenu au stade A ci-dessus, puis chauffe à 60°C pendant 1 heure le milieu réactionnel. On refroidit, ajoute une solution aqueuse saturée en chlorure de sodium, extrait à l'éther, sèche et concentre à sec sous pression réduite. On obtient 14 g de produit brut que l'on purifie par chromatographie sur silice (éluant : acétate d'éthyle-triéthylamine 97-3). F = 120°C.

### Stade C : trans (±) 5,6-dichloro 2,3-dihydro N-méthyl 2-(1-pyrrolidinyl) 1H-indèn 1-amine.

On refroidit à -20°C, 5 g de produit préparé au stade ci-dessus, 40 cm³ de tétrahydrofuranne et 4 cm³ de triéthylamine et ajoute goutte à goutte 17 cm³ de chlorure de méthanesulfonyle en solution dans 3 cm³ de tétrahydrofuranne. On agite 15 minutes à -20°C, laisse revenir à 0°C, ajoute 20 cm³ de méthylamine en solution dans l'éthanol, agite 22 heures et concentre à sec. On dilue à l'eau, ajoute quelques gouttes de lessive de soude, extrait à l'acétate d'éthyle, lave la phase organique à l'eau salée, sèche et élimine les solvants sous pression réduite. On obtient 5,7 g de produit que l'on purifie par chromatographie sur silice (éluant : acétate d'éthyle-méthanol-triéthylamine 80-10-10). On abandonne le résidu à 4°C et recueille 4,91 g de produit cristallisé. F ≃ 55°C.

### Stade D : trans (±) N-[5,6-dichloro 2,3-dihydro 2-(1-pyrrolidinyl) 1H-indèn-1-yl] N-méthyl 3-nitro benzène acétamide et son chlorhydrate.

On opère comme à l'exemple 1 en utilisant au départ 579 mg d'acide 3-nitrophényl acétique, 518 mg de carbonyldiimidazole dans 5 cm³ de tétrahydrofuranne et 800 mg de produit préparé au stade C dans 2 cm³ de tétrahydrofuranne. On obtient 1 g de produit sous forme de base puis 1 g de chlorhydrate brut que l'on recristallise dans un mélange isopropanol-méthanol puis dans l'éthanol. On obtient 563 mg de produit pur. F = 185-190°C.

| Analyse : C₂₂H₂₄Cl₃N₃O₃ | | | | | | | | |
|---|---|---|---|---|---|---|---|---|
| Calculé | C% | 54,5 | H% | 4,99 | N% | 8,67 | Cl% | 21,94 |
| Trouvé | | 54,4 | | 5,0 | | 8,6 | | 21,8 |

Le 5,6-dichloro 1H-indène utilisé au départ de l'exemple 8 a été préparé comme suit :

### Stade A : 5,6-dichloro 2,3-dihydro 1H-indèn 1-one.

On chauffe 30 minutes au reflux dans 100 cm³ de chlorure de thionyle 20 g d'acide 3-(3,4-dichlorophényl) propionique préparé comme indiqué dans J. Med.Chem. (1973) 16(2) 101-106. On évapore le chlorure de thionyle, ajoute au résidu 20 cm³ de chlorure de méthylène et verse dans une suspension comprenant 20 g de chlorure d'aluminium dans 150 cm³ de chlorure de méthylène. On agite 1 heure à température ambiante puis 30 minutes à 50°C, verse le milieu réactionnel sur de la glace, agite pendant 30 minutes, extrait au chlorure de méthylène, lave à l'eau, sèche, concentre à sec, reprend le résidu à l'éther, essore, sèche et obtient 80 g de produit attendu. F = 155°C.

### Stade B : 5,6-dichloro 2,3-dihydro 1H-indèn-1-ol.

On refroidit à 0°C 19,4 g du produit préparé au stade A en solution dans 285 cm³ de méthanol, ajoute 4,85 g de borohydrure de sodium, agite 1 heure, évapore le méthanol, dilue à l'eau, élimine l'excès d'hydrure par addition d'acide chlorhydrique et extrait à l'acétate d'éthyle. Après élimination des solvants sous pression réduite, on obtient 17,48 g de produit attendu. F = 84°C.

### Stade C : 5,6-dichloro 1H-indène.

On chauffe 2 heures au reflux 17,48 g du produit préparé au stade B dans 470 cm³ de toluène en présence de 1,63 g d'acide paratoluènesulfonique et 0,57 g de 4-terbutyl catéchol. On dilue à l'eau, ajoute 10 cm³ de soude N et extrait au toluène. On élimine le solvant sous pression réduite, reprend le résidu dans l'hexane, essore et sèche le produit cristallisé. On obtient 9 g de produit attendu. F = 76°C.

### EXEMPLE 9 : trans (±) 3,4-dichloro N-[2,3-dihydro 2-[méthyl (2-phényléthyl) amino] 1H-indèn-1-yl] N-méthyl benzène acétamide et son chlorhydrate.

### Stade A : trans (±) 2,3-dihydro 1-[méthyl (2-phényléthyl) amino] 1H-indèn-2-ol et son chlorhydrate.

On mélange 5,68 g de 1a,6a-dihydro 6H-indeno [1,2-b] oxirène dans 15 cm³ d'eau, ajoute 6,5 g de N-méthyl phénéthylamine et chauffe à 50-55°C pendant 1 heure. On laisse revenir à 20°C, sature en chlorure de sodium puis ajoute 1 cm³ de lessive de soude, extrait à l'acétate d'éthyle, lave à l'eau salée, sèche et élimine les solvants sous pression réduite. On obtient 13,21 g de produit attendu sous forme de base, que l'on dissout dans 45 cm³ d'éther, puis ajoute 15 cm³ d'une solution éthanolique d'acide chlorhydrique (6,6 N), amorce la cristallisation, abandonne 1 heure à température ambiante, essore et sèche sous pression réduite à 80°C et recueille 8,87 g du chlorhydrate attendu. F = 158-162°C.

### Stade B : trans (±) 2,3-dihydro N-méthyl 2-[méthyl (2-phényléthyl) amino] 1H-indèn-1-amine et son dichlorhydrate.

On opère comme à l'exemple 7 stade B en utilisant au départ 8,38 g de chlorhydrate obtenu au stade A et obtient 8,44 g de produit sous forme de base. On reprend 8,36 g de ce produit dans 30 cm³ d'éther éthylique et 20 cm³ d'éthanol et ajoute 10 cm³ d'une solution éthanolique de chlorure d'hydrogène, amorce la cristallisation, abandonne à 20°C, essore les cristaux, rince avec le mélange éthanol/éther (1-1) puis à l'éther, sèche à 80°C sous pression réduite et recueille 7,6 g de dichlorhydrate attendu que l'on recristallise dans l'isopropanol. F = 234-237°C. (décomp).

| Analyse : | | | | | | | | |
|---|---|---|---|---|---|---|---|---|
| Calculé | C% | 64,59 | H% | 7,42 | N% | 7,93 | Cl% | 20,07 |
| Trouvé | | 64,4 | | 7,4 | | 7,9 | | 20,0 |

### Stade C : trans (±) 3,4-dichloro N-[2,3-dihydro 2-[méthyl (2-phényléthyl) amino] 1H-indèn-1-yl] N-méthyl benzène acétamide et son chlorhydrate.

On opère comme à l'exemple 4 en utilisant 800 mg d'acide 3,4-dichlorophényl acétique, 632 mg de carbonyldiimidazole dans 8 cm³ de tétrahydrofuranne, 0,84 cm³ de triéthylamine puis 1,060 g de dichlorhydrate obtenu au stade B. On obtient 1,625 g de produit sous forme de base. 1,424 g de produit brut sont transformés en chlorhydrate à l'aide de 0,6 cm³ de solution éthanolique de chlorure d'hydrogène (6,6 N). On récupère 1,0 g de produit attendu. F = 200-202°C.

| Analyse : C₂₇H₂₈Cl₂N₂O, HCl | | | | | | | | |
|---|---|---|---|---|---|---|---|---|
| Calculé | C% | 64,36 | H% | 5,80 | N% | 5,56 | Cl% | 21,11 |
| Trouvé | | 64,2 | | 5,6 | | 5,5 | | 21,2 |

### EXEMPLE 10 : trans (±) 3,4-dichloro N-[2,3-dihydro 2-[(2-furylméthyl) méthylamino] 1H-indèn-1-yl] N-méthyl benzène acétamide et son butènedioate.

### Stade A : trans (±) 2,3-dihydro 1-[méthyl (2-furylméthyl) amino] 1H-indèn-2-ol et son chlorhydrate.

On opère comme à l'exemple 9 stade A en utilisant au départ 7 g de 1a,6a-dihydro 6H-indeno [1,2-b] oxirène dans 10 cm³ d'eau et 5,26 g de N-méthyl furylméthylamine. On obtient 9,86 g de produit sous forme de base que l'on chromatographie sur silice (éluant : acétate d'éthyle-chlorure de méthylène 4-6). 406 mg de l'huile obtenue sont ajoutés à 20 cm³ d'acétate d'éthyle et 0,5 cm³ de solution éthanolique de chlorure d'hydrogène (6,6 N). On obtient 412 mg de chlorhydrate attendu. F = 145-148°C.

| Analyse : C₁₅H₁₈O₂NCl | | | | | | | | |
|---|---|---|---|---|---|---|---|---|
| Calculé | C% | 64,40 | H% | 6,48 | N% | 5,00 | Cl% | 12,67 |
| Trouvé | | 64,4 | | 6,7 | | 4,9 | | 12,7 |

### Stade B : Chlorhydrate de trans (±) 2,3-dihydro N-méthyl 2-[méthyl(2-furylméthyl) amino] 1H-indèn-1-amine.

On opère comme à l'exemple 7 stade B en utilisant au départ 5,83 g de la base obtenue au stade A et obtient directement 3,69 g de chlorhydrate attendu que l'on recristallise dans l'éthanol. F = 180-184°C (décomp).

### Stade C: trans (±) 3,4-dichloro N-[2,3-dihydro 2-[(2-furylméthyl) méthylamino] 1H-indèn-1-yl] N-méthyl benzène acétamide et son butènedioate.

On opère comme à l'exemple 4 en utilisant 800 mg d'acide 3,4-dichlorophénylacétique, 632 mg de carbonyldiimidazole dans 8 cm³ de tétrahydrofuranne, 0,6 cm³ de triéthylamine puis 1,14 g de chlorhydrate obtenu au stade B. On obtient 1,606 g de produit sous forme de base. On dissout 1,490 g de base dans 10 cm³ d'acétate d'éthyle, ajoute à 20°C 348 mg d'acide maléique et chauffe pour obtenir la dissolution totale. On amorce la cristallisation à chaud, refroidit, essore à température ambiante, rince à l'acétate d'éthyle puis à l'éther, sèche à 20°C sous pression réduite et récupère 1,555 g de produit attendu que l'on recristallise dans l'éthanol. F = 154-156°C.

| Analyse : C₂₄H₂₄₄Cl₂N₂O = 559,45 | | | | | | | | |
|---|---|---|---|---|---|---|---|---|
| Calculé | C% | 60,11 | H% | 5,04 | N% | 5,01 | Cl% | 12,67 |
| Trouvé | | 60,1 | | 4,9 | | 4,7 | | 12,7 |

### EXEMPLE 11: trans (±) 3,4-dichloro N-[2,3-dihydro 2-[4-(2-méthoxyphényl) 1-pipérazinyl] 1H-indèn-1-yl] N-méthyl benzène acétamide et son chlorhydrate.

### Stade A : trans (±) 2,3-dihydro 1-[4-(2-méthoxyphényl) 1-pipérazinyl] 1H-indèn-2-ol et son chlorhydrate.

On opère comme à l'exemple 9 en utilisant 5,68 g de 1a,6a-dihydro 6H-indeno [1,2-b] oxirène dans 20 cm³ d'eau et 10 g de 1-(2′-méthoxyphényl) pipérazine. On obtient 17,76 g de produit sous forme de base que l'on chromatographie sur silice en éluant à l'acétate d'éthyle. On transforme 655 mg du produit purifié en chlorhydrate.Après recristallisation dans l'éthanol, on récupère 370 mg de chlorhydrate attendu. F = 152-154°C.

### Stade B : Chlorhydrate de trans (±) 2,3-dihydro N-méthyl 2-[4-(2-méthoxyphényl) 1-pipérazinyl] 1H-indèn-1-amine.

On opère comme à l'exemple 7B en utilisant au départ 9,58 g de produit sous forme de base préparé au stade A et obtient directement 3,49 g de chlorhydrate attendu. Après extraction des liqueurs mères à l'acétate d'éthyle et chromatographie sur silice (éluant : acétate d'éthyle-méthanol-triéthylamine 85-10-5), on obtient 5,81 g de base que l'on transforme en chlorhydrate. F = 246-250°C.

### Stade C : trans (±) 3,4-dichloro N-[2,3-dihydro 2-[4-(2-méthoxyphényl) 1-pipérazinyl] 1H-indèn-1-yl] N-méthyl benzène acétamide et son chlorhydrate.

On opère comme à l'exemple 4 en utilisant 800 mg d'acide 3,4-dichlorophénylacétique, 632 mg de carbonyldiimidazole dans 8 cm³ de tétrahydrofuranne, 1,01 g de chlorhydrate obtenu au stade B et 0,5 cm³ de triéthylamine. On obtient 1,94 g de produit sous forme de base dont on transforme 1,92 g en chlorhydrate. On récupère après recristallisation dans l'isopropanol, 1,08 g de chlorhydrate attendu. F = 217-220°C.

| Analyse : C₂₉H₃₁Cl₂N₃O₂ | | | | | | | | |
|---|---|---|---|---|---|---|---|---|
| Calculé | C% | 63,09 | H% | 5,75 | N% | 7,49 | Cl% | 18,96 |
| Trouvé | | 62,1 | | 5,9 | | 7,2 | | 19,1 |

### Exemple 12 :

On à préparé des comprimés répondant à la formule suivante :

| | |
|---|---|
| - Produit de l'exemple 1 | 200 mg |
| - Excipient q.s.p. | 800 mg |

(Détail de l'excipient : lactose, talc, amidon, stéarate de magnésium).

### EXEMPLE 13 :

On a préparé un soluté injectable (voie intra-musculaire) répondant à la formule suivante :

| | |
|---|---|
| - Produit de l'exemple 1 | 50 mg |
| - Solvant stérile q.s.p. | 5 cm³ |

### ETUDE PHARMACOLOGIQUE

### 1) Mesure de l'activité diurétique.

Des rats mâles, de souche Sprague Dawley et de poids 180-200 g sont mis à jeun 17 heures avant l'essai tout en recevant de l'eau ad libitum.

Des lots de 10 animaux sont constitués par dose testée, les rats reçoivent le produit à tester, par voie intraveineuse, en solution dans du sérum physiologique à 0,9 % de chlorure de sodium sous un volume de 1 ml/kg.

Le volume urinaire est mesuré 1 heure après l'administration du produit. Les urines sont recueillies et l'activité du produit est exprimée en pourcentage de variation calculé sur le volume urinaire correspondant des témoins.

On obtient les résultats suivants :

| | Dose en mg/kg | Pourcentage de variation du volume urinaire |
|---|---|---|
| Produit de l'exemple 1 | 0,3 | + 1530 % |
| | 1 | + 3180 % |
| Produit de l'exemple 9 de la demande européenne EP 0.258.096 | 0,3 | + 850 % |
| | 1 | + 1680 % |

Conclusion : le pourcentage d'augmentation du volume urinaire obtenu, avec le produit de l'exemple 1 de la présente demande à la dose de 0,3 mg/kg est comparable à celui obtenu avec le produit racémique décrit dans la demande européenne EP 0.258.096 à une dose de 1 mg/kg.

L'activité diurétique du produit de la présente demande ne s'accompagne pas de modification de l'élimination des électrolytes. Il s'agit donc essentiellement d'une diurèse aqueuse.

## Revendications (Revendications pour l'(les) Etat(s) contractant(s) suivant(s): AT, BE, CH, DE, FR, GB, IT, LI, LU, NL, SE)

1. Composés de formule (I) : 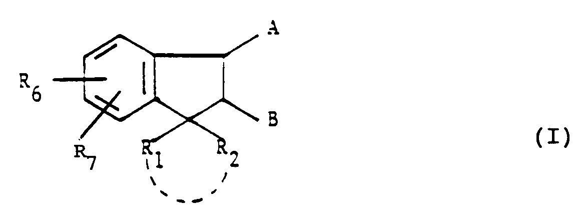 dans laquelle R₁ et R₂, identiques ou différents, représentent un atome d'hydrogène ou un radical alkyle renfermant de 1 à 5 atomes de carbone ou R₁ et R₂ forment ensemble avec l'atome de carbone auquel ils sont liés un cycle cycloalkyle renfermant entre 3 et 6 atomes de carbones et pouvant contenir éventuellement un hétéroatome tel qu'un atome d'oxygène ou de soufre ou d'azote et ou bien R₆ et R₇, identiques ou différents, représentent un atome d'halogène, un radical alkyle ou alcoxy renfermant de 1 à 5 atomes de carbone et A et B sont tels que l'un des substituants A ou B est choisi dans
- le groupe de formule 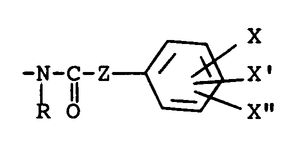 R étant un atome d'hydrogène ou un radical alkyle renfermant de 1 à 5 atomes de carbone, Z une chaîne alkylène linéaire - (CH₂)ₙ-, n étant un nombre entier pouvant varier entre 0 et 5 ou une chaîne alkylène ramifiée renfermant de 2 à 8 atomes de carbone ou un groupement -CH₂-O-, X, X' et X'', identiques ou différents, représentent un atome d'hydrogène, un radical alkyle ou alkoxy renfermant de 1 à 4 atomes de carbone, un atome d'halogène, un radical hydroxyle, trifluorométhyle, nitro, amino, monoalkyle ou dialkylamino ou sulfoamino et l'autre des substituants A ou B est choisi dans
- le groupe de formule 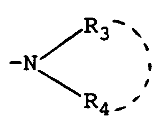 R₃ et R₄, identiques ou différents, représentent un atome d'hydrogène ou un radical alkyle renfermant de 1 à 5 atomes de carbone ou R₃ et R₄ forment ensemble avec l'atome d'azote auquel ils sont liés un hétérocycle à 5 ou 6 chaînons comportant éventuellement un autre hétéroatome tel que l'oxygène, l'azote ou le soufre, l'atome d'azote étant éventuellement substitué par un radical alkyle renfermant de 1 à 4 atomes de carbone,
ou bien R₆ et R₇ représentent un atome d'hydrogène et
- soit B représente un radical pyrrolidine et A représente un groupe 3,5-bis (1,1-diméthyléthyl) 4-hydroxy N-méthyl benzamide, N-méthyl 3-nitrobenzène acétamide ou 3,4-dichloro N-méthyl benzène acétamide, ces deux derniers produits étant nécessairement sous forme d'énantiomères purs,
- soit A représente un radical 3,4-dichloro N-méthyl benzène acétamide et B représente un groupe méthyl (2-phényléthyl) amino ou (2-furylméthyl) méthylamino ou 4-(2-méthoxyphényl) 1-pipérazinyle,
ou bien R₆ et R₇ sont tels que l'un représente un atome d'hydrogène l'autre un atome de chlore, B représente un radical pyrrolidine et A représente un groupe N-méthyl 3-nitro benzène acétamide,
lesdits composés de formule (I) pouvant être sous toutes leurs formes énantiomères ou diastéréoisomères possibles ainsi que leurs hydrates et les sels d'addition avec les acides de ces composés et de leurs hydrates, ainsi que le trans (±) 3,4-dichloro N-[2,3-dihydro 2-(2,5-dihydro 1H-pyrrol-1-yl) 1H-indèn-1-yl] N-méthyl benzène acétamide sous forme de base libre ou de chlorhydrate.

2. Composés de formule (I), tels que définis à la revendication 1, caractérisés en ce que les groupements A et B sont en configuration trans ainsi que leurs hydrates et les sels d'addition avec les acides.

3. Composés de formule (I) , tels que définis à la revendica tion 1 ou 2, caractérisés en ce que dans le groupe 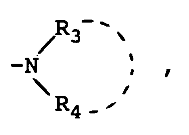 R₃ et R₄ forment avec l'atome d'azote auquel ils sont liés l'hétérocycle pyrrolidine, ainsi que leurs hydrates et leurs sels d'addition avec les acides.

4. Composés de formule (I), tels que définis à l'une quelconque des revendications 1 à 3, caractérisés en ce que le groupe 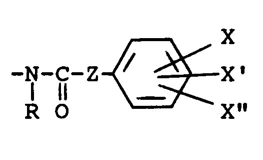 représente 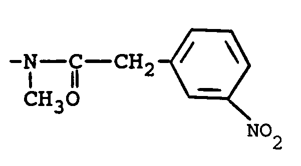 ainsi que leurs hydrates et leurs sels d'addition avec les acides.

5. Composés de formule (I), tels que définis à l'une quelconque des revendications 1 à 4, caractérisés en ce que R₁ et R₂ représentent tous deux un atome d'hydrogène, ainsi que leurs hydrates et leurs sels d'addition avec les acides.

6. Composés de formule (I), tels que définis à l'une quelconque des revendications 1 à 5, caractérisés en ce que R₆ et R₇, identiques ou différents représentent un atome d'hydrogène ou de chlore, ainsi que leurs hydrates et leurs sels d'addition avec les acides.

7. Le (1S,2S) N-[2,3-dihydro 2-(1-pyrrolidinyl) 1H-indèn-1-yl] N-méthyl 3-nitro benzène acétamide et son chlorhydrate anhydre.

8. Le trans (±) N-[5,6-dichloro 2,3-dihydro 2-(1-pyrrolidinyl) 1H-indèn-1-yl] N-méthyl 3-nitro benzène acétamide et son chlorhydrate.

9. Le trans (±) N-[5-chloro 2,3-dihydro 2-(1-pyrrolidinyl) 1H-indèn-1-yl] N-méthyl 3-nitro benzène acétamide et son chlorhydrate.

10. Procédé de préparation des produits de formule (I) dont les groupements A et B sont en configuration trans, caractérisé en ce que l'on condense le produit de formule (II) : 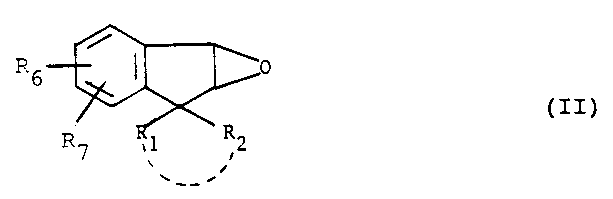 R₆, R₇, R₁ et R₂ ayant toutes les significations données précédemment,
- soit avec une amine de formule (III) : 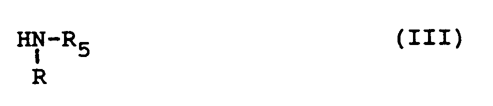 R ayant toutes les significations données précédemment et R₅ étant un groupement protecteur de la fonction amine et notamment un groupement benzyle, pour obtenir un produit de formule (IV) : 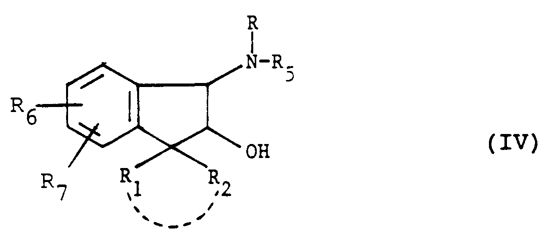 dont on active la fonction hydroxyle et que l'on condense avec une amine de formule (V) : 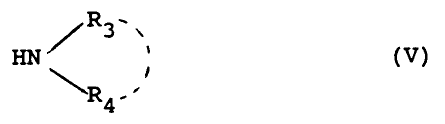 dans laquelle R₃ et R₄ ont les significations données précédemment pour obtenir un produit de formule (VI) : 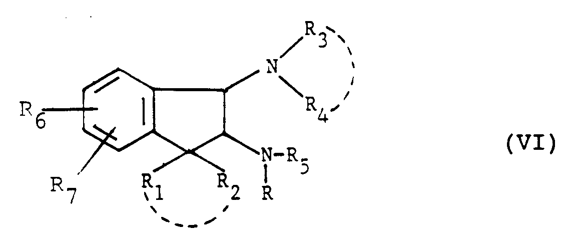 dont on élimine le groupement protecteur R₅ de la fonction amine pour obtenir un produit de formule (VII) : 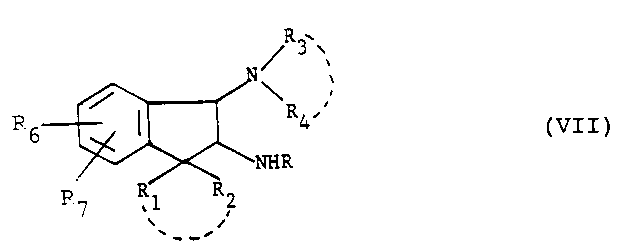 que l'on traite avec un acide de formule (VIII) ou un dérivé fonctionnel de cet acide : 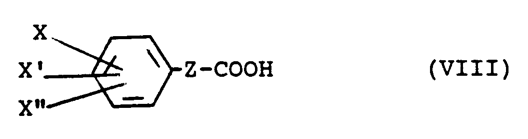 dans laquelle Z, X, X' et X'' ont toutes les significations précédentes pour obtenir un produit de formule (I) dans laquelle A représente le groupe 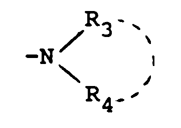 et B le groupe 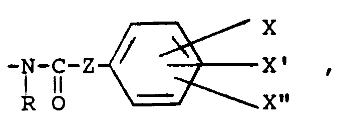
- soit avec une amine de formule : 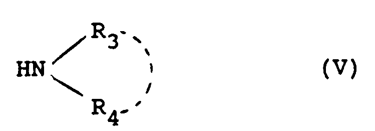 pour obtenir un produit de formule (IX) : 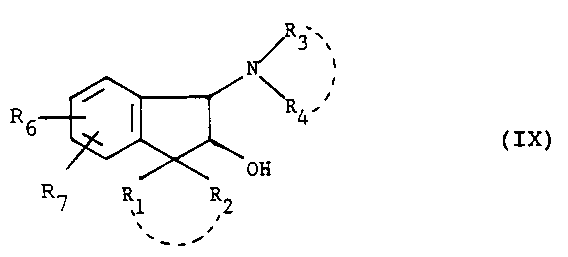 dont on active la fonction hydroxyle et que l'on traite avec une amine de formule (X) :
NH₂R (X)
dans laquelle R a la signification précédente pour obtenir un produit de formule (XI) : 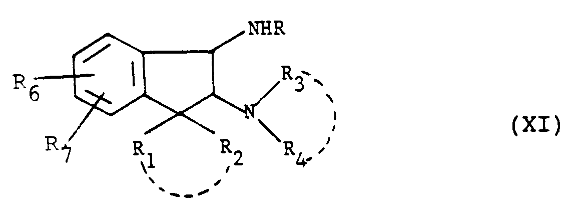 que l'on condense avec un acide de formule (VIII) ou un dérivé fonctionnel de cet acide: 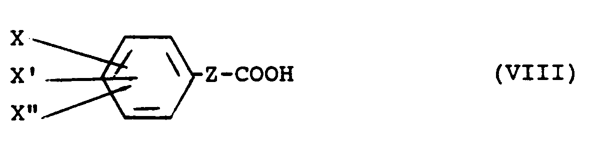 dans laquelle Z, X, X' et X'' ont les significations précédentes pour obtenir un produit de formule (I) dans laquelle A représente le groupe 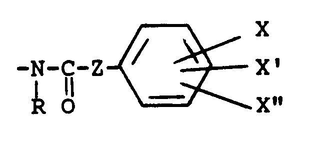 et B le groupe 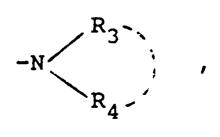 lesdits composés de formule (I) pouvant être dédoublés pour obtenir les formes optiquement actives et que l'on traite, si désiré, avec un acide minéral ou organique, pour obtenir un sel.

11. A titre de médicaments, les produits de formule (I), tels que définis à l'une quelconque des revendications 1 à 6, ainsi que leurs hydrates et les sels d'addition avec les acides pharmaceutiquement acceptables.

12. A titre de médicaments, les produits de la revendication 7, 8 ou 9, pharmaceutiquement acceptables.

13. Les compositions pharmaceutiques contenant à titre de principe actif, l'un au moins des médicaments tels que définis à la revendication 11 ou 12.

14. A titre de produits intermédiaires, les produits de formule (IV), (VI), (VII), (IX), (XI) dans laquelle R₆ et R₇ ne représentent pas tous deux un atome d'hydrogène.

## Revendications (Revendications pour l'(les) Etat(s) contractant(s) suivant(s): ES, GR)

1. Procédé pour préparer les composés de formule (I) : 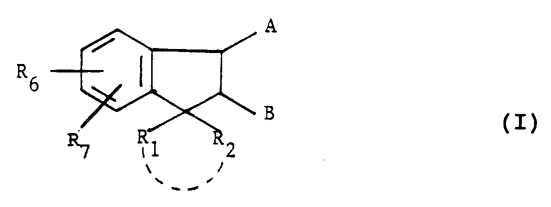 dans laquelle R₁ et R₂, identiques ou différents, représentent un atome d'hydrogène ou un radical alkyle renfermant de 1 à 5 atomes de carbone ou R₁ et R₂ forment ensemble avec l'atome de carbone auquel ils sont liés un cycle cycloalkyle renfermant entre 3 et 6 atomes de carbones et pouvant contenir éventuellement un hétéroatome tel qu'un atome d'oxygène ou de soufre ou d'azote et ou bien R₆ et R₇, identiques ou différents, représentent un atome d'halogène, un radical alkyle ou alcoxy renfermant de 1 à 5 atomes de carbone et A et B sont tels que l'un des substituants A ou B est choisi dans
- le groupe de formule 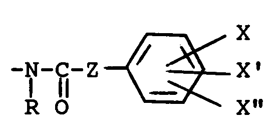 R étant un atome d'hydrogène ou un radical alkyle renfermant de 1 à 5 atomes de carbone, Z une chaîne alkylène linéaire - (CH₂)ₙ-, n étant un nombre entier pouvant varier entre 0 et 5 ou une chaîne alkylène ramifiée renfermant de 2 à 8 atomes de carbone ou un groupement -CH₂-O-, X, X' et X'', identiques ou différents, représentent un atome d'hydrogène, un radical alkyle ou alkoxy renfermant de 1 à 4 atomes de carbone, un atome d'halogène, un radical hydroxyle, trifluorométhyle, nitro, amino, monoalkyle ou dialkylamino ou sulfoamino et l'autre des substituants A ou B est choisi dans
- le groupe de formule 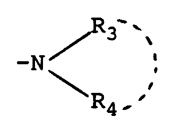 R₃ et R₄, identiques ou différents, représentent un atome d'hydrogène ou un radical alkyle renfermant de 1 à 5 atomes de carbone ou R₃ et R₄ forment ensemble avec l'atome d'azote auquel ils sont liés un hétérocycle à 5 ou 6 chaînons comportant éventuellement un autre hétéroatome tel que l'oxygène, l'azote ou le soufre, l'atome d'azote étant éventuellement substitué par un radical alkyle renfermant de 1 à 4 atomes de carbone,
ou bien R₆ et R₇ représentent un atome d'hydrogène et
- soit B représente un radical pyrrolidine et A représente un groupe 3,5-bis (1,1-diméthyléthyl) 4-hydroxy N-méthyl benzamide, N-méthyl 3-nitrobenzène acétamide ou 3,4-dichloro N-méthyl benzène acétamide, ces deux derniers produits étant nécessairement sous forme d'énantiomères purs,
- soit A représente un radical 3,4-dichloro N-méthyl benzène acétamide et B représente un groupe méthyl (2-phényléthyl) amino ou (2-furylméthyl) méthylamino ou 4-(2-méthoxyphényl) 1-pipérazinyle,
ou bien R₆ et R₇ sont tels que l'un représente un atome d'hydrogène l'autre un atome de chlore, B représente un radical pyrrolidine et A représente un groupe N-méthyl 3-nitro benzène acétamide,
lesdits composés de formule (I) pouvant être sous toutes leurs formes énantiomères ou diastéréoisomères possibles ainsi que leurs hydrates et les sels d'addition avec les acides de ces composés et de leurs hydrates, ainsi que le trans (±) 3,4 dichloro N-[2,3-dihydro 2-(2,5-dihydro 1H-pyrrol-1-yl) 1H-indèn-1-yl] N-méthyl benzène acétamide sous forme de base libre ou de chlorhydrate, caractérisé en ce que l'on condense le produit de formule (II) : 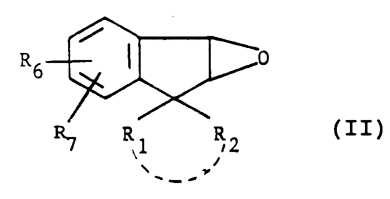 R₆, R₇, R₁ et R₂ ayant toutes les significations données précédemment,
- soit avec une amine de formule (III) : 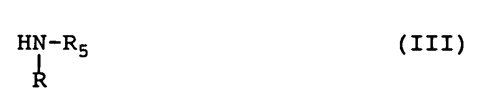 R ayant toutes les significations données précédemment et R₅ étant un groupement protecteur de la fonction amine et notamment un groupement benzyle, pour obtenir un produit de formule (IV) : 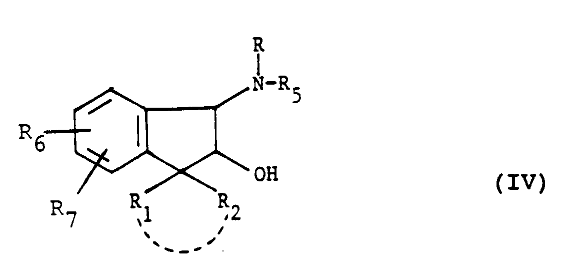 dont on active la fonction hydroxyle et que l'on condense avec une amine de formule (V) : 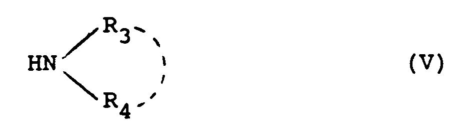 dans laquelle R₃ et R₄ ont les significations données précédemment pour obtenir un produit de formule (VI) : 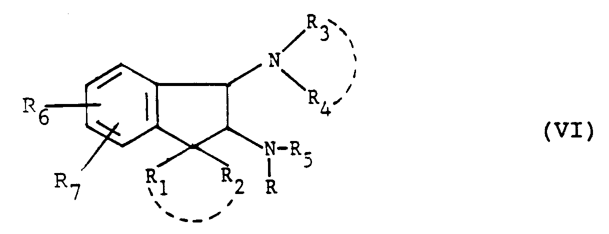 dont on élimine le groupement protecteur R₅ de la fonction amine pour obtenir un produit de formule (VII) : 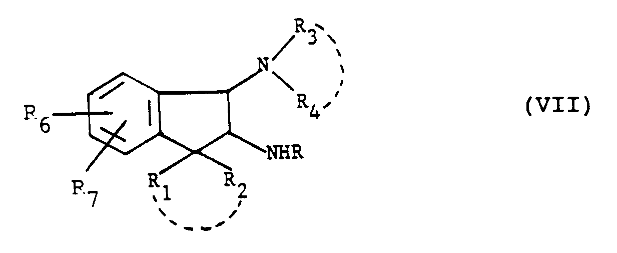 que l'on traite avec un acide de formule (VIII) ou un dérivé fonctionnel de cet acide : 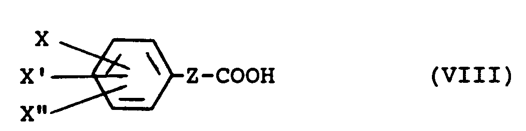 dans laquelle Z, X, X' et X'' ont toutes les significations précédentes pour obtenir un produit de formule (I) dans laquelle A représente le groupe 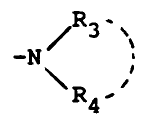 et B le groupe 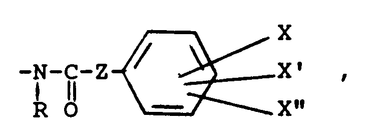
- soit avec une amine de formule : 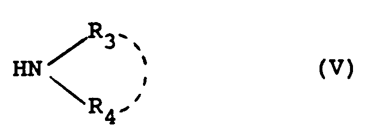 pour obtenir un produit de formule (IX) : 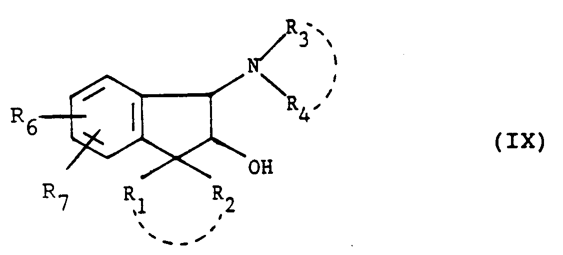 dont on active la fonction hydroxyle et que l'on traite avec une amine de formule (X) :
NH₂R (X)
dans laquelle R a la signification précédente pour obtenir un produit de formule (XI) : 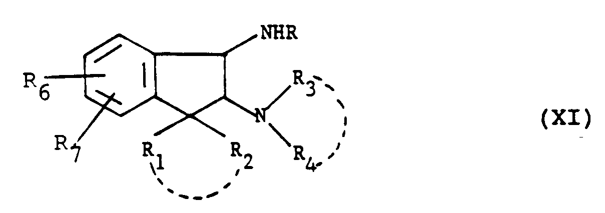 que l'on condense avec un acide de formule (VIII) ou un dérivé fonctionnel de cet acide : 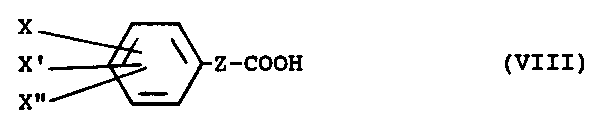 dans laquelle Z, X, X' et X'' ont les significations précédentes pour obtenir un produit de formule (I) dans laquelle A représente le groupe 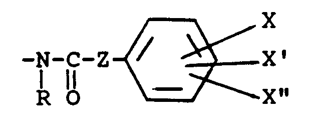 et B le groupe 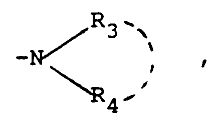 lesdits composés de formule (I) pouvant être dédoublés pour obtenir les formes optiquement actives et que l'on traite, si désiré, avec un acide minéral ou organique, pour obtenir un sel.

2. Procédé selon la revendication 1, caractérisé en ce que l'on utilise au départ une amine de formule (V) dans laquelle R₃ et R₄ forment avec l'atome d'azote auquel ils sont liés l'hétérocycle pyrrolidine.

3. Procédé selon la revendication 1 ou 2, caractérisé en ce que l'on utilise au départ une amine de formule (III) ou (X) dans laquelle R représente un radical CH₃ et un acide de formule (VIII) ou un dérivé fonctionnel de cet acide de formule

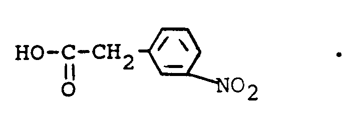

4. Procédé selon l'une quelconque des revendications 1 à 3, caractérisé en ce que l'on utilise au départ un produit de formule (II) dans laquelle R₁ et R₂ représentent tous deux un atome d'hydrogène.

5. Procédé selon l'une quelconque des revendications 1 à 4, caractérisé en ce que l'on utilise au départ un produit de formule (II) dans laquelle R₆ et R₇ identiques représentent un atome d'hydrogène.

6. Procédé selon l'une quelconque des revendications 1 à 4, caractérisé en ce que l'on utilise au départ un produit de formule (II) dans laquelle R₆ et R₇ identiques représentent un atome de chlore.

7. Procédé selon l'une quelconque des revendications 1 à 5, caractérisé en ce que l'on prépare le (1S,2S) N-[2,3-dihydro 2-(1-pyrrolidinyl) 1H-indèn-1-yl] N-méthyl 3-nitro benzène acétamide et son chlorhydrate anhydre.

8. Procédé selon l'une quelconque des revendications 1 à 4 ou 6, caractérisé en ce que l'on prépare le trans (±) N-[5,6-dichloro 2,3-dihydro 2-(1-pyrrolidinyl) 1H-indèn-1-yl] N-méthyl 3-nitro benzène acétamide et son chlorhydrate.

9. Procédé de préparation de compositions pharmaceutiques, caractérisé en ce que l'on met à titre de principe actif l'un au moins des composés de formule (I) tels que définis à la revendication 1 ou l'un au moins de leurs hydrates ou des sels d'addition avec les acides pharmaceutiquement acceptables de ces produits ou de leurs hydrates sous une forme destinée à cet usage.

10. Procédé de préparation de compositions pharmaceutiques, caractérisé en ce que l'on met à titre de principe actif l'un au moins des composés de formule (I) tels que définis à l'une quelconque des revendications 2 à 6 ou l'un au moins de leurs hydrates ou des sels d'addition avec les acides pharmaceutiquement acceptables de ces produits ou de leurs hydrates sous une forme destinée à cet usage.

11. Procédé de préparation de compositions pharmaceutiques, caractérisé en ce que l'on met à titre de principe actif le (1S,2S) N-[2,3-dihydro 2-(1-pyrrolidinyl) 1H-indèn-1-yl] N-méthyl 3-nitro benzène acétamide et son chlorhydrate anhydre ou l'un de ses hydrates ou sels pharmaceutiquement acceptables sous une forme destinée à cet usage.

12. Procédé de préparation de compositions pharmaceutiques, caractérisé en ce que l'on met à titre de principe actif le trans (±) N-(5,6-dichloro 2,3-dihydro 2-(1-pyrrolidinyl) 1H-indèn-1-yl] N-méthyl 3-nitro benzène acétamide ou l'un de ses hydrates ou sels pharmaceutiquement acceptables sous une forme destinée à cet usage.

## Claims (Claims for the following Contracting State(s): AT, BE, CH, DE, FR, GB, IT, LI, LU, NL, SE)

1. The compounds of formula (I): 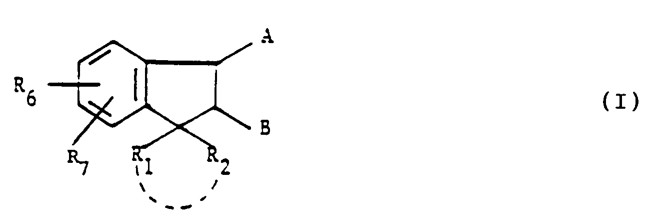 in which R₁ and R₂, identical or different, represent a hydrogen atom or an alkyl radical containing 1 to 5 carbon atoms or R₁ and R₂ form together with the carbon atom to which they are linked a cycloalkyl radical containing between 3 and 6 carbon atoms and able to optionally contain a heteroatom such as an oxygen or sulphur or nitrogen atom and either R₆ and R₇, identical or different, represent a halogen atom, an alkyl or alkoxy radical containing 1 to 5 carbon atoms and A and B are such that one of the substituents A or B is chosen from
- the group of formula 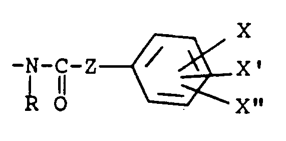 R being a hydrogen atom or an alkyl radical containing 1 to 5 carbon atoms, Z a linear alkylene chain -(CH₂)ₙ-, n being an integer which can vary between 0 and 5 or a branched alkylene chain containing 2 to 8 carbon atoms or a -CH₂-O- group, X, X' and X'', identical or different, represent a hydrogen atom, an alkyl or alkoxy radical containing 1 to 4 carbon atoms, a halogen atom, a hydroxyl, trifluoromethyl, nitro, amino, monoalkyl or dialkylamino or sulphoamino radical and the other of the substituents A or B is chosen from
- the group of formula 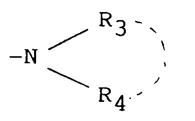 R₃ and R₄, identical or different, represent a hydrogen atom or an alkyl radical containing 1 to 5 carbon atoms or R₃ and R₄ form together with the nitrogen atom to which they are linked a heterocycle with 5 or 6 links optionally containing another heteroatom such as oxygen, nitrogen or sulphur, the nitrogen atom being optionally substituted by an alkyl radical containing 1 to 4 carbon atoms,
or R₆ and R₇ represent a hydrogen atom and
- either B represents a pyrrolidine radical and A represents a 3,5-bis (1,1-dimethylethyl) 4-hydroxy N-methyl benzamide group, an N-methyl 3-nitrobenzene acetamide group or a 3,4-dichloro N-methyl benzene acetamide group, these last two products being necessarily in the form of pure enantiomers,
- or A represents a 3,4-dichloro N-methyl benzene acetamide radical and B represents a methyl (2-phenylethyl) amino or (2-furylmethyl) methylamino or 4-(2-methoxyphenyl) 1-piperazinyl group,
or R₆ and R₇ are such that one represents a hydrogen atom and the other represents a chlorine atom, B represents a pyrrolidine radical and A represents an N-methyl 3-nitro benzene acetamide group,
the said compounds of formula (I) being in all their possible enantiomeric or diastereoisomeric forms as well as their hydrates and the addition salts with acids of these compounds and their hydrates, as well as trans (±) 3,4-dichloro N-[2,3-dihydro 2-(2,5-dihydro 1H-pyrrol-1-yl) 1H-inden-1-yl] N-methyl benzene acetamide in the form of the free base or the hydrochloride.

2. The compounds of formula (I) as defined in claim 1, characterized in that the groups A and B are in trans configuration as well as their hydrates and the addition salts with acids.

3. The compounds of formula (I) as defined in claim 1 or 2, characterized in that in the 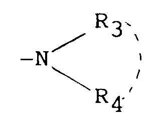 group, R₃ and R₄ form with the nitrogen atom to which they are linked the pyrrolidine heterocycle, as well as their hydrates and their addition salts with acids.

4. The compounds of formula (I) as defined in any one of claims 1 to 3, characterized in that the 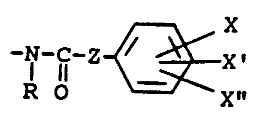 group represents 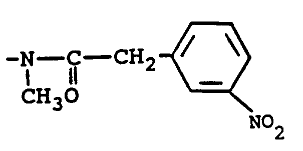 as well as their hydrates and their addition salts with acids.

5. The compounds of formula (I) as defined in any one of claims 1 to 4, characterized in that R₁ and R₂ both represent a hydrogen atom, as well as their hydrates and their addition salts with acids.

6. The compounds of formula (I) as defined in any one of claims 1 to 5, characterized in that R₆ and R₇, identical or different, represent a hydrogen or chlorine atom, as well as their hydrates and their addition salts with acids.

7. (1S,2S) N-[2,3-dihydro 2-(1-pyrrolidinyl) 1H-inden-1-yl] N-methyl 3-nitro benzene acetamide and its anhydrous hydrochloride.

8. trans (±) N-[5,6-dichloro 2,3-dihydro 2-(1-pyrrolidinyl) 1H-inden-1-yl] N-methyl 3-nitro benzene acetamide and its hydrochloride.

9. trans (±) N-[5-chloro 2,3-dihydro 2-(1-pyrrolidinyl) 1H-inden-1-yl] N-methyl 3-nitro benzene acetamide and its hydrochloride.

10. Preparation process for the products of formula (I), the A and B groups of which are in trans configuration, characterized in that the product of formula (II): 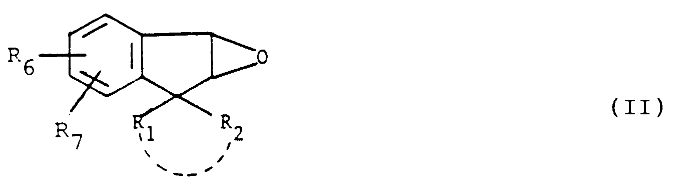 R₆, R₇, R₁ and R₂ having any of the meanings given previously, is condensed
- either with an amine of formula (III): 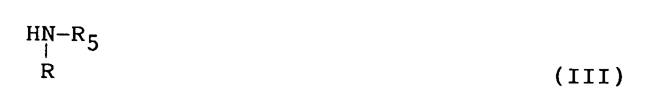 R having any of the meanings given previously and R₅ being a protective group of the amine function and in particular a benzyl group, in order to obtain a product of formula (IV): 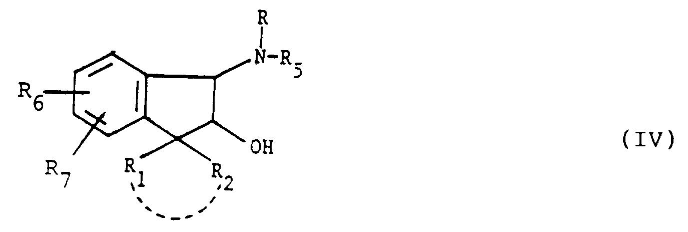 the hydroxyl function of which is activated and which is condensed with an amine of formula (V): 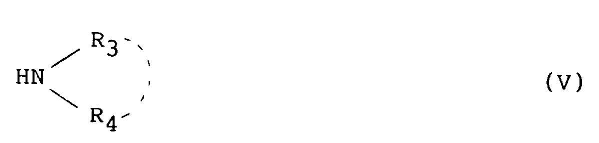 in which R₃ and R₄ have the meanings given previously in order to obtain a product of formula (VI): 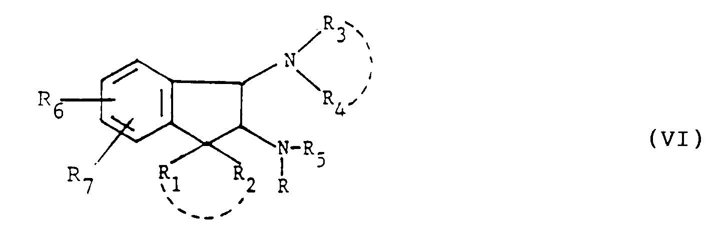 of which the protective group R₅ of the amine function is eliminated in order to obtain a product of formula (VII): 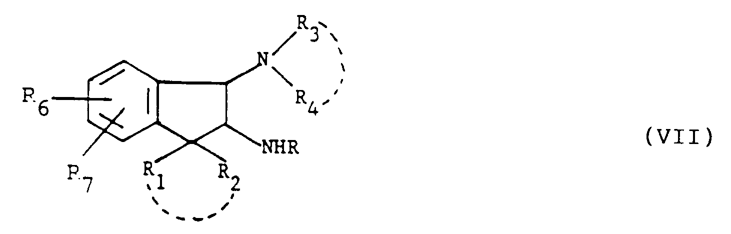 which is treated with an acid of formula (VIII) or a functional derivative of this acid: 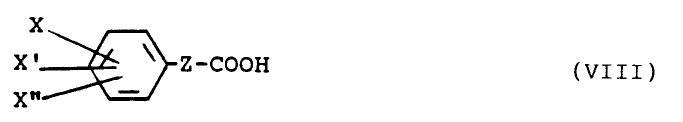 in which Z, X, X' and X'' have any of the previous meanings in order to obtain a product of formula (I) in which A represents the 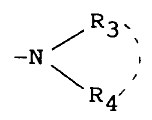 group and B the 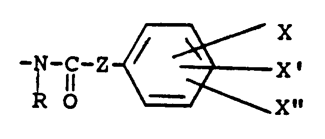 group,
- or with an amine of formula: 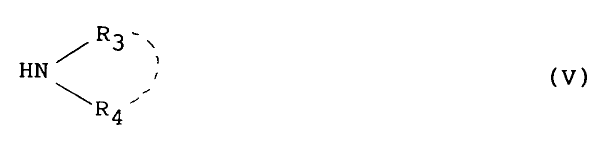 in order to obtain a product of formula (IX): 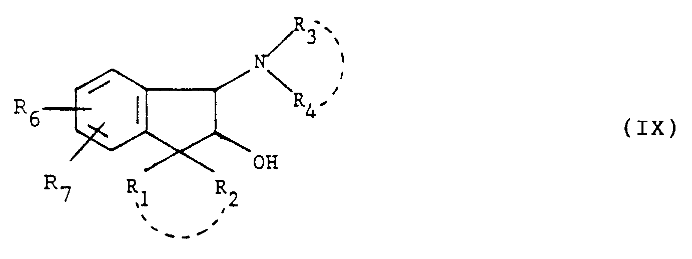 the hydroxyl function of which is activated and which is treated with an amine of formula (X):
NH₂R (X)
in which R has the previous meaning in order to obtain a product of formula (XI): 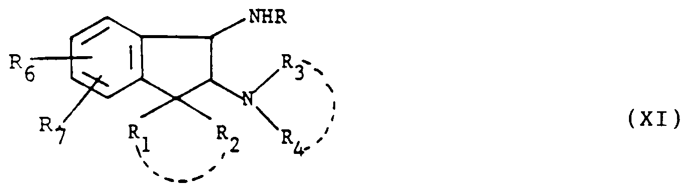 which is condensed with an acid of formula (VIII) or a functional derivative of this acid: 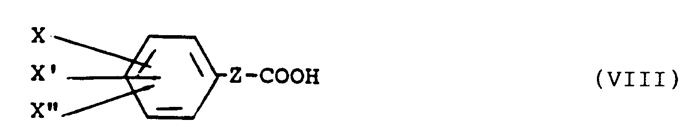 in which Z, X, X' and X'' have the previous meanings in order to obtain a product of formula (I) in which A represents the 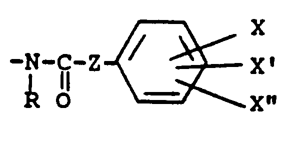 group and B the 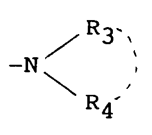 group, the said compounds of formula (I) being able to be resolved in order to obtain the optically active forms and, if desired, treated with a mineral or organic acid in order to obtain a salt.

11. As medicaments, the products of formula (I) as defined in any one of claims 1 to 6, as well as their hydrates and the addition salts with pharmaceutically acceptable acids.

12. As medicaments, the pharmaceutically acceptable products of claim 7, 8 or 9.

13. The pharmaceutical compositions containing as active ingredient at least one of the medicaments defined in claim 11 or 12.

14. As intermediate products, the products of formula (IV), (VI), (VII), (IX), (XI) in which R₆ and R₇ do not both represent a hydrogen atom.

## Claims (Claims for the following Contracting State(s): ES, GR)

1. Preparation process for the compounds of formula (I): 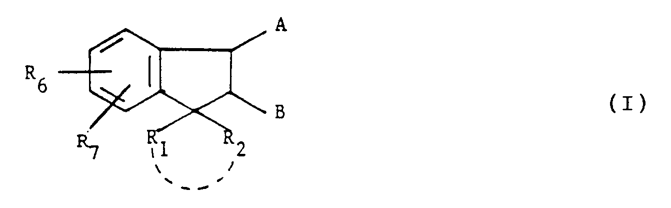 in which R₁ and R₂, identical or different, represent a hydrogen atom or an alkyl radical containing 1 to 5 carbon atoms or R₁ and R₂ form together with the carbon atom to which they are linked a cycloalkyl radical containing between 3 and 6 carbon atoms and able to optionally contain a heteroatom such as an oxygen or sulphur or nitrogen atom and either R₆ and R₇, identical or different, represent a halogen atom, an alkyl or alkoxy radical containing 1 to 5 carbon atoms and A and B are such that one of the substituents A or B is chosen from
- the group of formula 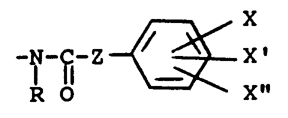 R being a hydrogen atom or an alkyl radical containing 1 to 5 carbon atoms, Z a linear alkylene chain -(CH₂)ₙ-, n being an integer which can vary between 0 and 5 or a branched alkylene chain containing 2 to 8 carbon atoms or a -CH₂-O- group, X, X' and X'', identical or different, represent a hydrogen atom, an alkyl or alkoxy radical containing 1 to 4 carbon atoms, a halogen atom, a hydroxyl, trifluoromethyl, nitro, amino, monoalkyl or dialkylamino or sulphoamino radical and the other of the substituents A or B is chosen from
- the group of formula 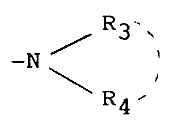 R₃ and R₄, identical or different, represent a hydrogen atom or an alkyl radical containing 1 to 5 carbon atoms or R₃ and R₄ form together with the nitrogen atom to which they are linked a heterocycle with 5 or 6 links optionally containing another heteroatom such as oxygen, nitrogen or sulphur, the nitrogen atom being optionally substituted by an alkyl radical containing 1 to 4 carbon atoms,
or R₆ and R₇ represent a hydrogen atom and
- either B represents a pyrrolidine radical and A represents a 3,5-bis (1,1-dimethylethyl) 4-hydroxy N-methyl benzamide group, an N-methyl 3-nitrobenzene acetamide group or a 3,4-dichloro N-methyl benzene acetamide group, these last two products being necessarily in the form of pure enantiomers,
- or A represents a 3,4-dichloro N-methyl benzene acetamide radical and B represents a methyl (2-phenylethyl) amino or (2-furylmethyl) methylamino or 4-(2-methoxyphenyl) 1-piperazinyl group,
or R₆ and R₇ are such that one represents a hydrogen atom and the other represents a chlorine atom, B represents a pyrrolidine radical and A represents an N-methyl 3-nitro benzene acetamide group,
the said compounds of formula (I) being in all their possible enantiomeric or diastereoisomeric forms as well as their hydrates and the addition salts with acids of these compounds and their hydrates, as well as trans (±) 3,4-dichloro N-[2,3-dihydro 2-(2,5-dihydro 1H-pyrrol-1-yl) 1H-inden-1-yl] N-methyl benzene acetamide in the form of the free base or the hydrochloride, characterized in that the product of formula (II): 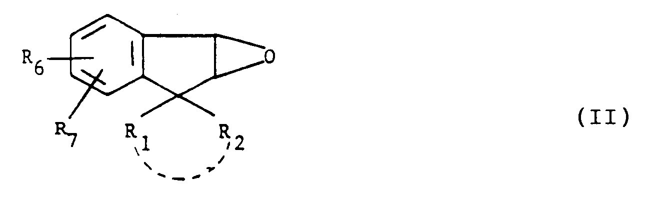 R₆, R₇, R₁ and R₂ having any of the meanings given previously, is condensed
- either with an amine of formula (III): 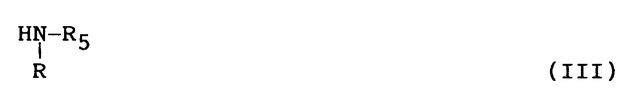 R having any of the meanings given previously and R₅ being a protective group of the amine function and in particular a benzyl group, in order to obtain a product of formula (IV): 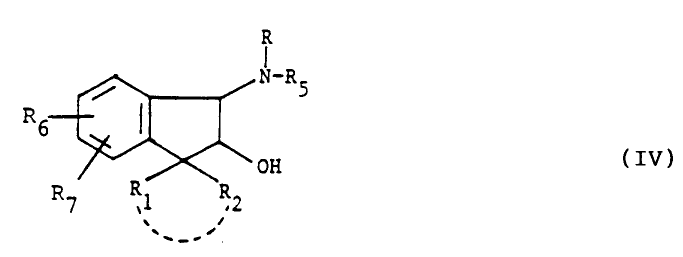 the hydroxyl function of which is activated and which is condensed with an amine of formula (V): 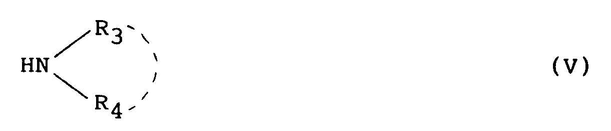 in which R₃ and R₄ have the meanings given previously in order to obtain a product of formula (VI): 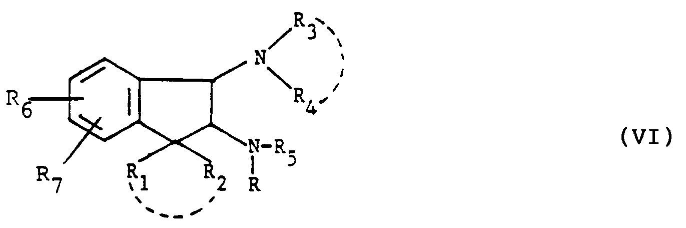 of which the protective group R₅ of the amine function is eliminated in order to obtain a product of formula (VII): 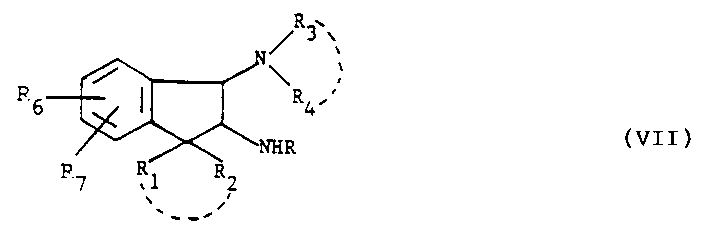 which is treated with an acid of formula (VIII) or a functional derivative of this acid: 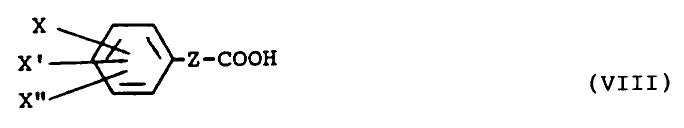 in which Z, X, X' and X'' have any of the previous meanings in order to obtain a product of formula (I) in which A represents the 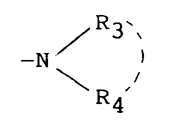 group and B the 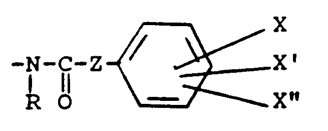 group,
- or with an amine of formula: 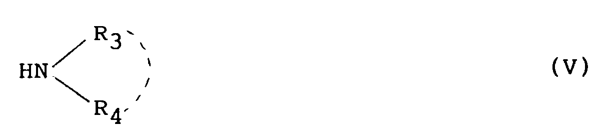 in order to obtain a product of formula (IX): 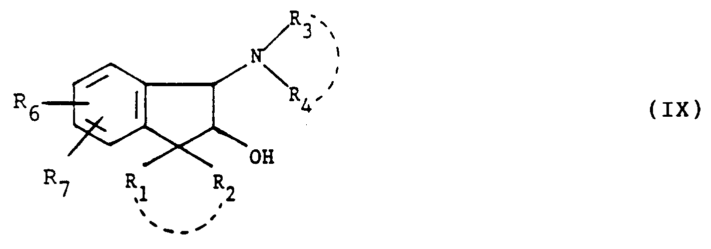 the hydroxyl function of which is activated and which is treated with an amine of formula (X):
NH₂R (X)
in which R has the previous meaning in order to obtain a product of formula (XI): 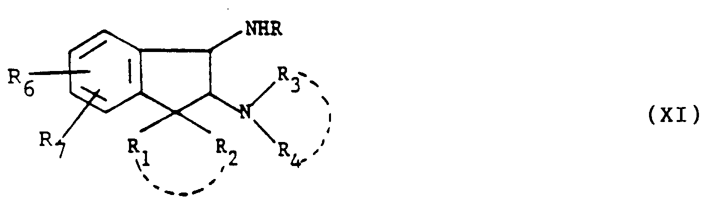 which is condensed with an acid of formula (VIII) or a functional derivative of this acid: 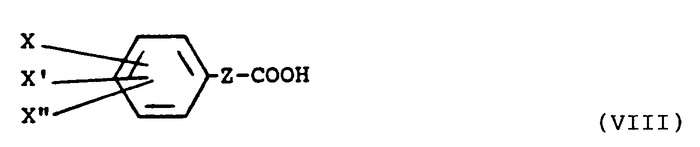 in which Z, X, X' and X'' have the previous meanings in order to obtain a product of formula (I) in which A represents the 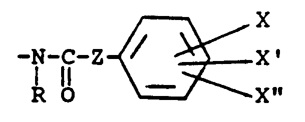 group and B the 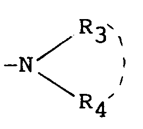 group, the said compounds of formula (I) being able to be resolved in order to obtain the optically active forms and, if desired, treated with a mineral or organic acid in order to obtain a salt.

2. Process according to claim 1, characterized in that an amine of formula (V) is used at the start in which R₃ and R₄ form together with the nitrogen atom to which they are linked the pyrrolidine heterocycle.

3. Process according to claim 1 or 2, characterized in that an amine of formula (III) or (X) in which R represents a CH₃ radical and an acid of formula (VIII) or a functional derivative of this acid of formula 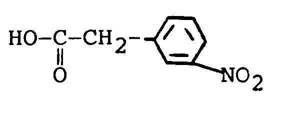 are used a the start.

4. Process according to any one of claims 1 to 3, characterized in that a product of formula (II) is used at the start in which R₁ and R₂ both represent a hydrogen atom.

5. Process according to any one of claims 1 to 4, characterized in that a product of formula (II) is used at the start in which R₆ and R₇ are identical and represent a hydrogen atom.

6. Process according to any one of claims 1 to 4, characterized in that a product of formula (II) is used at the start in which R₆ and R₇ are identical and represent a chlorine atom.

7. Process according to any one of claims 1 to 5, characterized in that (1S,2S) N-[2,3-dihydro 2-(1-pyrrolidinyl) 1H-inden-1-yl] N-methyl 3-nitro benzene acetamide and its anhydrous hydrochloride are prepared.

8. Process according to any one of claims 1 to 4 or 6, characterized in that trans (±) N-[5,6-dichloro 2,3-dihydro 2-(1-pyrrolidinyl) 1H-inden-1-yl] N-methyl 3-nitro benzene acetamide and its hydrochloride are prepared.

9. Preparation process for pharmaceutical compositions, characterized in that at least one of the compounds of formula (I) as defined in claim 1 or at least one of their hydrates or the addition salts with pharmaceutically acceptable acids of these products or their hydrates is used as active ingredient in a form intended for this use.

10. Preparation process for pharmaceutical compositions, characterized in that at least one of the compounds of formula (I) as defined in any one of claims 2 to 6 or at least one of their hydrates or the addition salts with pharmaceutically acceptable acids of these products or their hydrates is used as active ingredient in a form intended for this use.

11. Preparation process for pharmaceutical compositions, characterized in that (1S,2S) N-[2,3-dihydro 2-(1-pyrrolidinyl) 1H-inden-1-yl] N-methyl 3-nitro benzene acetamide and its hydrochloride or one of its hydrates or pharmaceutically acceptable salts are used as active ingredient in a form intended for this use.

12. Preparation process for pharmaceutical compositions, characterized in that trans (±) N-[5,6-dichloro 2,3-dihydro 2-(1-pyrrolidinyl) 1H-inden-1-yl] N-methyl 3-nitro benzene acetamide or one of its hydrates or pharmaceutically acceptable salts is used as active ingredient in a form intended for this use.

## Patentansprüche (Patentansprüche für folgende(n) Vertragsstaat(en): AT, BE, CH, DE, FR, GB, IT, LI, LU, NL, SE)

1. Verbindungen der Formel (I) 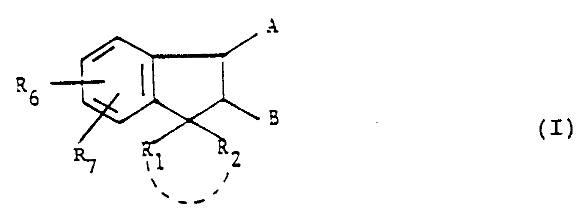 worin R₁ und R₂, identisch oder verschieden, ein Wasserstoffatom oder einen Alkylrest mit 1 bis 5 Kohlenstoffatomen bedeuten, oder R₁ und R₂ gemeinsam mit dem Kohlenstoffatom, an das sie gebunden sind, einen Cycloalkylring bilden, der zwischen 3 und 6 Kohlenstoffatome enthält und gegebenenfalls ein Heteroatom wie ein Sauerstoff- oder Schwefel- oder Stickstoffatom enthalten kann, und entweder R₆ und R₇, identisch oder verschieden, ein Halogenatom, einen Alkyl- oder Alkoxyrest mit 1 bis 5 Kohlenstoffatomen bedeuten, und A und B derart sind, daß einer der Substituenten A oder B ausgewählt ist unter
- der Gruppe der Formel 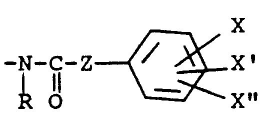 worin R ein Wasserstoffatom oder eine Alkylgruppe mit 1 bis 5 Kohlenstoffatomen bedeutet, Z eine lineare Alkylenkette -(CH₂)ₙ-, in der n eine ganze Zahl darstellt, die zwischen 0 und 5 variieren kann, oder eine verzweigte Alkylenkette mit 2 bis 8 Kohlenstoffatomen oder eine Gruppe -CH₂-O- darstellt, X, X' und X'', identisch oder verschieden, ein Wasserstoffatom, einen Alkyl- oder Alkoxyrest mit 1 bis 4 Kohlenstoffatomen, ein Halogenatom, eine Hydroxy-, Trifluormethyl-, Nitro-, Amino- Monoalkyl- oder Dialkylamino- oder Sulfoaminogruppe darstellen, und
der andere der Substituenten A oder B ausgewählt ist unter
- der Gruppe der Formel 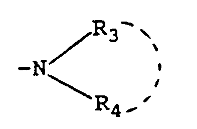 worin R₃ und R₄, identisch oder verschieden, ein Wasserstoffatom oder einen Alkylrest mit 1 bis 5 Kohlenstoffatomen darstellen, oder R₃ und R₄ gemeinsam mit dem Stickstoffatom, an das sie gebunden sind, einen Heterocyclus mit 5 oder 6 Kettengliedern bilden, der gegebenenfalls ein weiteres Heteroatom wie Sauerstoff, Stickstoff oder Schwefel enthält, wobei das Stickstoffatom gegebenenfalls durch einen Alkylrest mit 1 bis 4 Kohlenstoffatomen substituiert ist.
oder R₆ und R₇ ein Wasserstoffatom wiedergeben, und
- entweder B einen Pyrrolidinrest darstellt, und A eine 3,5-Bis-(1,1-dimethylethyl)-4-hydroxy-N-methylbenzamid-, N-Methyl-3-nitrobenzolacetamid- oder 3,4-Dichlor-N-methylbenzolacetamidgruppe darstellt, wobei diese beiden letztgenannten Produkte zwangsläufig in Form reiner Enantiomerer vorliegen,
- oder A einen 3,4-Dichlor-N-methylbenzolacetamidrest bedeutet, und B eine Methyl-(2-phenylethyl)-amino- oder (2-Furylmethyl)-methylamino- oder 4-(2-Methoxyphenyl)-1-piperazinylgruppe wiedergibt,
oder R₆ und R₇ derart sind, daß eines ein Wasserstoffatom, das andere ein Chloratom bedeutet, B einen Pyrrolidinrest darstellt, und A für eine N-Methyl-3-nitrobenzolacetamidgruppe steht,
wobei die Verbindungen der Formel (I) in sämtlichen ihrer möglichen enantiomeren oder diastereomeren Formen vorliegen können, sowie deren Hydrate und die Additionssalze dieser Verbindungen mit Säuren und deren Hydrate, sowie das (±)-3,4-Dichlor-N-[2,3-dihydro-2-(2,5-dihydro-1H-pyrrol-1-yl)-1H-inden-1-yl]-N-methylbenzolacetamid in Form der freien Base oder des Hydrochlorids.

2. Verbindungen der Formel (I), wie in Anspruch 1 definiert, dadurch gekennzeichnet, daß die Gruppen A und B in der trans-Konfiguration vorliegen, sowie deren Hydrate und Additionssalze mit Säuren.

3. Verbindungen der Formel (I), wie in Anspruch 1 oder 2 definiert, dadurch gekennzeichnet, daß in der Gruppe 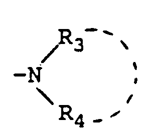 R₃ und R₄ mit dem Stickstoffatom, an das sie gebunden sind, den Pyrrolidinheterocyclus bilden, sowie deren Hydrate und deren Additionssalze mit Säuren.

4. Verbindungen der Formel (I), wie in einem der Ansprüche 1 bis 3 definiert, dadurch gekennzeichnet, daß die Gruppe 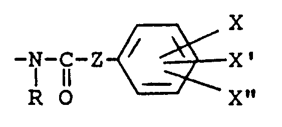 für 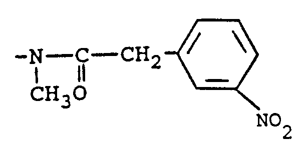 steht, sowie deren Hydrate und deren Additionssalze mit Säuren.

5. Verbindungen der Formel (I) gemäß einem der Ansprüche 1 bis 4, dadurch gekennzeichnet, daß R₁ und R₂ beide ein Wasserstoffatom bedeuten, sowie deren Hydrate und deren Additionssalze mit Säuren.

6. Verbindungen der Formel (I) gemäß einem der Ansprüche 1 bis 5, dadurch gekennzeichnet, daß R₆ und R₇, identisch oder verschieden, ein Wasserstoff- oder Chloratom bedeuten, sowie deren Hydrate und deren Additionssalze mit Säuren.

7. (1S,2S)-N-[2,3-Dihydro-2-(1-pyrrolidinyl)-1H-inden-1-yl]-N-methyl-3-nitrobenzolacetamid und dessen wasserfreies Hydrochlorid.

8. Trans-(±)-N-[5,6-dichlor-2,3-dlhydro-2-(1-pyrrolidinyl)-1H-inden-1-yl]-N-methyl-3-nitrobenzolacetamid und dessen Hydrochlorid.

9. Trans-(±)-N-[5-chlor-2,3-dihydro-2-(1-pyrrolidinyl)-1H-inden-1-yl]-N-methyl-3-nitrobenzolacetamid und dessen Hydrochlorid.

10. Verfahren zur Herstellung der Produkte der Formel (I), deren Gruppen A und B sich in der trans-Konfiguration befinden, dadurch gekennzeichnet, daß man das Produkt der Formel (II) 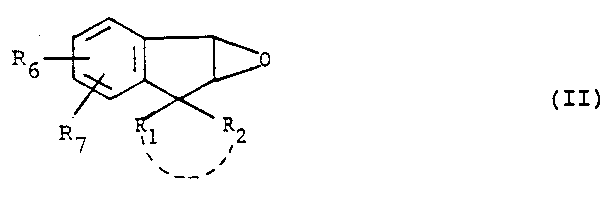 worin R₆, R₇, R₁ und R₂ sämtliche der vorstehenden Bedeutungen besitzen,
- entweder mit einem Amin der Formel (III) 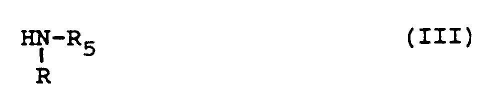 worin R sämtliche der vorstehend angegebenen Bedeutungen besitzt, und R₅ eine Schutzgruppe für die Aminfunktion und insbesondere eine Benzylgruppe ist, kondensiert, um zu einem Produkt der Formel (IV) 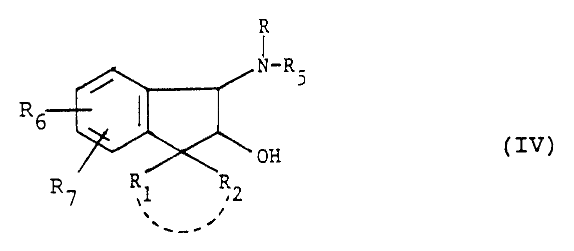 zu gelangen, dessen Hydroxylfunktion man aktiviert und welches man mit einem Amin der Formel (V) 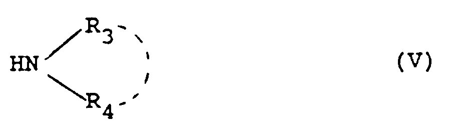 worin R₃ und R₄ die vorstehend angegebenen Bedeutungen besitzen, kondensiert, um zu einem Produkt der Formel (VI) 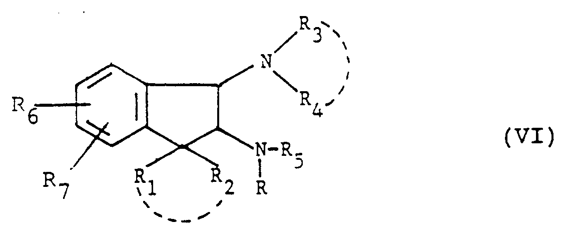 zu gelangen, an dem man die Schutzgruppe R₅ der Aminfunktion entfernt, um zu einem Produkt der Formel (VII) 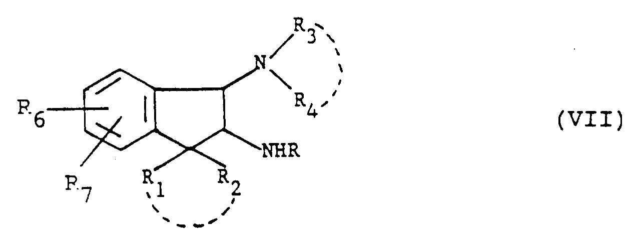 zu gelangen, welches man mit einer Säure der Formel (VIII) oder einem funktionellen Derivat dieser Säure 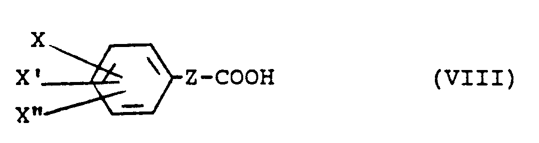 worin Z, X, X' und X'' sämtliche vorstehenden Bedeutungen besitzen, behandelt, um zu einem Produkt der Formel (I) zu gelangen, worin A für die Gruppe 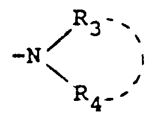 und B für die Gruppe 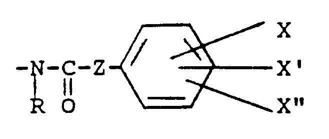 steht,
- oder mit einem Amin der Formel 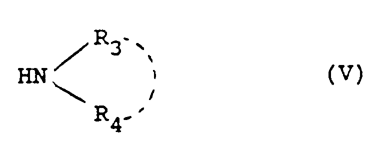 kondensiert, um zu einem Produkt der Formel (IX) 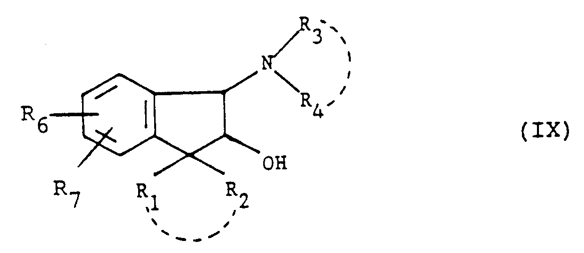 zu gelangen, dessen Hydroxylfunktion man aktiviert und das man mit einem Amin der Formel (X)
NH₂R (X)
worin R die vorstehende Bedeutung besitzt, behandelt, um zu einem Produkt der Formel (XI) 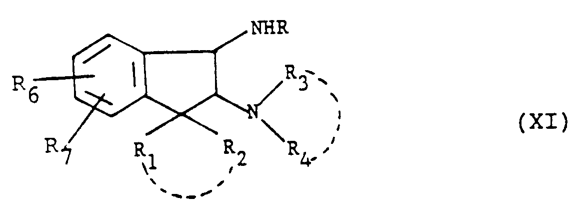 zu gelangen, das man mit einer Säure der Formel (VIII) oder einem funktionellen Derivat dieser Säure 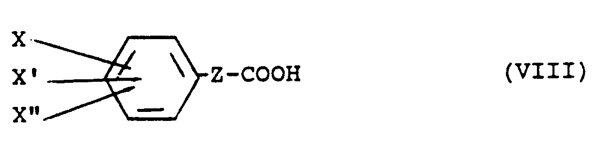 worin Z, X, X' und X'' die vorstehenden Bedeutungen besitzen, kondensiert, um zu einem Produkt der Formel (I) zu gelangen, worin A für die Gruppe 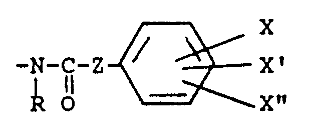 und B für die Gruppe 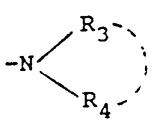 steht, wobei die Verbindungen der Formel (I) aufgetrennt werden können, um die optisch aktiven Formen zu erhalten, und das man gewünschtenfalls mit einer Mineralsäure oder organischen Säure behandelt, um ein Salz zu erhalten.

11. Als Arzneimittel die Produkte der Formel (I), wie in einem der Ansprüche 1 bis 6 definiert, sowie deren Hydrate und die Additionssalze mit pharmazeutisch verträglichen Säuren.

12. Als Arzneimittel die pharmazeutisch verträglichen Produkte gemäß Anspruch 7, 8 oder 9.

13. Pharmazeutische Zusammensetzungen, enthaltend als Wirkstoff zumindest eines der Arzneimittel, wie in Anspruch 11 oder 12 definiert.

14. Als Zwischenprodukte die Produkte der Formel (IV), (VI), (VII), (IX), (XI), worin R₆ und R₇ nicht alle beide ein Wasserstoffatom bedeuten.

## Patentansprüche (Patentansprüche für folgende(n) Vertragsstaat(en): ES, GR)

1. Verfahren zur Herstellung der Verbindungen der Formel (I) 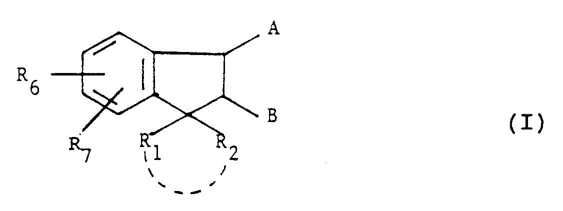 worin R₁ und R₂, identisch oder verschieden, ein Wasserstoffatom oder einen Alkylrest mit 1 bis 5 Kohlenstoffatomen bedeuten, oder R₁ und R₂ gemeinsam mit dem Kohlenstoffatom, an das sie gebunden sind, einen Cycloalkylring bilden, der zwischen 3 und 6 Kohlenstoffatome enthält und gegebenenfalls ein Heteroatom wie ein Sauerstoff- oder Schwefel- oder Stickstoffatom enthalten kann, und
entweder R₆ und R₇, identisch oder verschieden, ein Halogenatom, einen Alkyl- oder Alkoxyrest mit 1 bis 5 Kohlenstoffatomen bedeuten, und A und B derart sind, daß einer der Substituenten A oder B ausgewählt ist unter
- der Gruppe der Formel 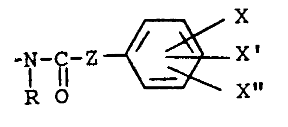 worin R ein Wasserstoffatom oder eine Alkylgruppe mit 1 bis 5 Kohlenstoffatomen bedeutet, Z eine lineare Alkylenkette -(CH₂)ₙ-, in der n eine ganze Zahl darstellt, die zwischen 0 und 5 variieren kann, oder eine verzweigte Alkylenkette mit 2 bis 8 Kohlenstoffatomen oder eine Gruppe -CH₂-O- darstellt, X, X' und X'', identisch oder verschieden, ein Wasserstoffatom, einen Alkyl- oder Alkoxyrest mit 1 bis 4 Kohlenstoffatomen, ein Halogenatom, eine Hydroxy-, Trifluormethyl-, Nitro-, Amino- Monoalkyl- oder Dialkylamino- oder Sulfoaminogruppe darstellen, und
der andere der Substituenten A oder B ausgewählt ist unter
- der Gruppe der Formel 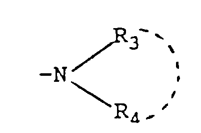 worin R₃ und R₄, identisch oder verschieden, ein Wasserstoffatom oder einen Alkylrest mit 1 bis 5 Kohlenstoffatomen darstellen, oder R₃ und R₄ gemeinsam mit dem Stickstoffatom, an das sie gebunden sind, einen Heterocyclus mit 5 oder 6 Kettengliedern bilden, der gegebenenfalls ein weiteres Heteroatom wie Sauerstoff, Stickstoff oder Schwefel enthält, wobei das Stickstoffatom gegebenenfalls durch einen Alkylrest mit 1 bis 4 Kohlenstoffatomen substituiert ist.
oder R₆ und R₇ ein Wasserstoffatom wiedergeben, und
- entweder B einen Pyrrolidinrest darstellt, und A eine 3,5-Bis-(1,1-dimethylethyl)-4-hydroxy-N-methylbenzamid-, N-Methyl-3-nitrobenzolacetamid- oder 3,4-Dichlor-N-methylbenzolacetamidgruppe darstellt, wobei diese beiden letztgenannten Produkte zwangsläufig in Form reiner Enantiomerer vorliegen,
- oder A einen 3,4-Dichlor-N-methylbenzolacetamidrest bedeutet, und B eine Methyl-(2-phenylethyl)-amino- oder (2-Furylmethyl)-methylamino- oder 4-(2-Methoxyphenyl)-1-piperazinylgruppe wiedergibt,
oder R₆ und R₇ derart sind, daß eines ein Wasserstoffatom, das andere ein Chloratom bedeutet, B einen Pyrrolidinrest darstellt, und A für eine N-Methyl-3-nitrobenzolacetamidgruppe steht,
wobei die Verbindungen der Formel (I) in sämtlichen ihrer möglichen enantiomeren oder diastereomeren Formen vorliegen können, sowie von deren Hydraten und den Additionssalzen dieser Verbindungen mit Säuren und deren Hydraten, sowie des trans-(±)-3,4-Dichlor-N-[2,3-dihydro-2-(2,5-dihydro-1H-pyrrol-1-yl)-1H-inden-1-yl]-N-methylbenzolacetamids in Form der freien Base oder des Hydrochlorids, dadurch gekennzeichnet, daß man das Produkt der Formel 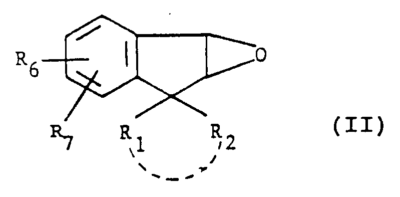 worin R₆, R₇, R₁ und R₂ sämtliche der vorstehenden Bedeutungen besitzen,
- entweder mit einem Amin der Formel (III) 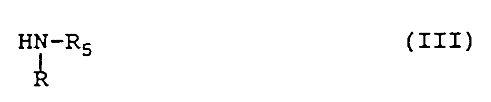 worin R sämtliche der vorstehend angegebenen Bedeutungen besitzt, und R₅ eine Schutzgruppe für die Aminfunktion und insbesondere eine Benzylgruppe ist, kondensiert, um zu einem Produkt der Formel (IV) 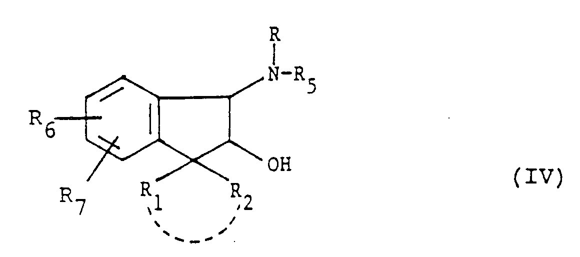 zu gelangen, dessen Hydroxylfunktion man aktiviert und welches man mit einem Amin der Formel (V) 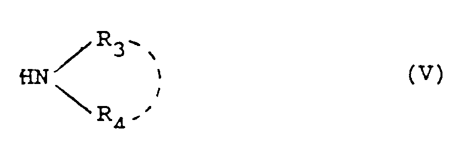 worin R₃ und R₄ die vorstehend angegebenen Bedeutungen besitzen, kondensiert, um zu einem Produkt der Formel (VI) 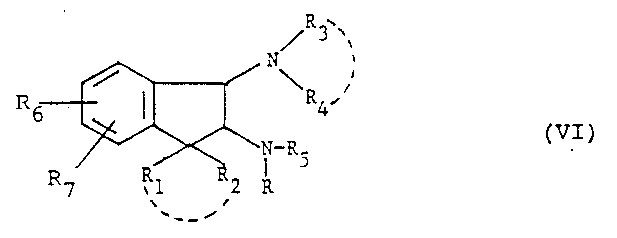 zu gelangen, an dem man die Schutzgruppe R₅ der Aminfunktion entfernt, um zu einem Produkt der Formel (VII) 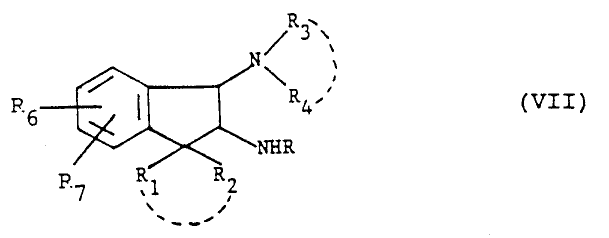 zu gelangen, welches man mit einer Säure der Formel (VIII) oder einem funktionellen Derivat dieser Säure 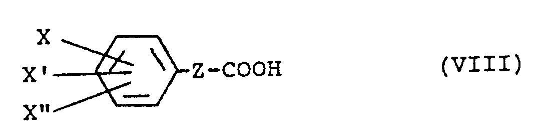 worin Z, X, X' und X'' sämtliche vorstehenden Bedeutungen besitzen, behandelt, um zu einem Produkt der Formel (I) zu gelangen, worin A für die Gruppe 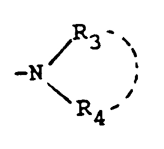 und B für die Gruppe 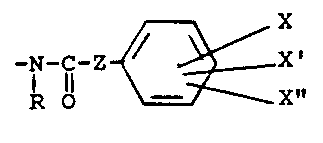 steht,
- oder mit einem Amin der Formel 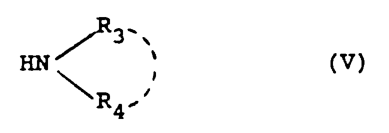 kondensiert, um zu einem Produkt der Formel (IX) 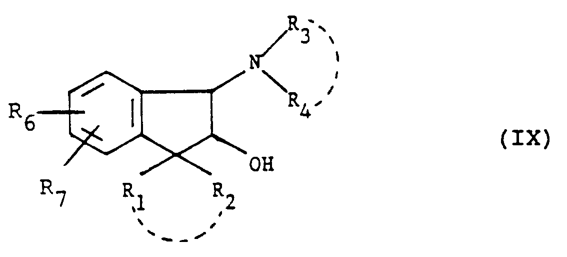 zu gelangen, dessen Hydroxylfunktion man aktiviert und das man mit einem Amin der Formel (X)
NH₂R (X)
worin R die vorstehende Bedeutung besitzt, behandelt, um zu einem Produkt der Formel (XI) 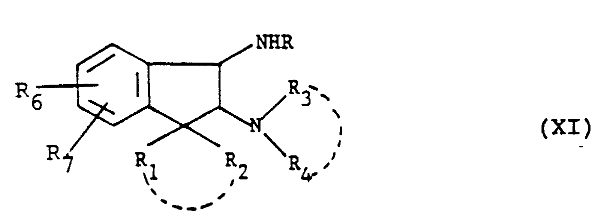 zu gelangen, das man mit einer Säure der Formel (VIII) oder einem funktionellen Derivat dieser Säure 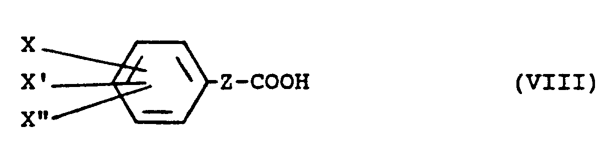 worin Z, X, X' und X'' die vorstehenden Bedeutungen besitzen, kondensiert, um zu einem Produkt der Formel (I) zu gelangen, worin A für die Gruppe 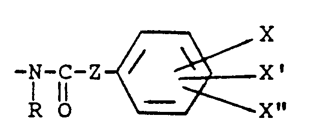 und B für die Gruppe 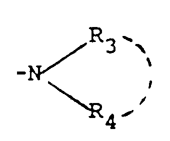 steht, wobei die Verbindungen der Formel (I) aufgetrennt werden können, um die optisch aktiven Formen zu erhalten, und das man gewünschtenfalls mit einer Mineralsäure oder organischen Säure behandelt, um ein Salz zu erhalten.

2. Verfahren gemäß Anspruch 1, dadurch gekennzeichnet, daß man von einem Amin der Formel (V) ausgeht, worin R₃ und R₄ mit dem Stickstoffatom, an das sie gebunden sind, einen Pyrrolidinheterocyclus bilden.

3. Verfahren gemäß Anspruch 1 oder 2, dadurch gekennzeichnet, daß man von einem Amin der Formel (III) oder (X), worin R einen Rest CH₃ darstellt, und einer Säure der Formel (VIII) oder einem funktionellen Derivat dieser Säure der Formel 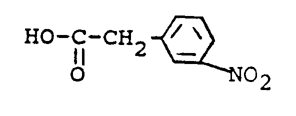 ausgeht.

4. Verfahren gemäß einem der Ansprüche 1 bis 3, dadurch gekennzeichnet, daß man von einem Produkt der Formel (II) ausgeht, worin R₁ und R₂ alle beide ein Wasserstoffatom bedeuten.

5. Verfahren gemäß einem der Ansprüche 1 bis 4, dadurch gekennzeichnet, daß man von einem Produkt der Formel (II) ausgeht, worin R₆ und R₇, die identisch sind, ein Wasserstoffatom bedeuten.

6. Verfahren gemäß einem der Ansprüche 1 bis 4, dadurch gekennzeichnet, daß man von einem Produkt der Formel (II) ausgeht, worin R₆ und R₇, die identisch sind, ein Chloratom bedeuten.

7. Verfahren gemäß einem der Ansprüche 1 bis 5, dadurch gekennzeichnet, daß man das (1S,2S)-N-[2,3-Dihydro-2-(1-pyrrolidinyl)-1H-inden-1-yl]-N-methyl-3-nitrobenzolacetamid und sein wasserfreies Hydrochlorid herstellt.

8. Verfahren gemäß einem der Ansprüche 1 bis 4 oder 6, dadurch gekennzeichnet, daß man das trans-(±)-N-[5,6-Dichlor-2,3-dihydro-2-(1-pyrrolidinyl)-1H-inden-1-yl]-N-methyl-3-nitrobenzolacetamid und sein Hydrochlorid herstellt.

9. Verfahren zur Herstellung von pharmazeutischen Zusammensetzungen, dadurch gekennzeichnet, daß man als Wirkstoff zumindest eine der Verbindungen der Formel (I), wie in Anspruch 1 definiert, oder zumindest eines ihrer Hydrate oder Additionssalze dieser Produkte mit pharmazeutisch verträglichen Säuren oder deren Hydrate in eine für diese Verwendung bestimmte Form bringt.

10. Verfahren zur Herstellung von pharmazeutischen Zusammensetzungen, dadurch gekennzeichnet, daß man als Wirkstoff zumindest eine der Verbindungen der Formel (I), wie in einem der Ansprüche 2 bis 6 definiert, oder zumidest eines ihrer Hydrate oder die Additionssalze dieser Produkte mit pharmazeutisch verträglichen Säuren oder deren Hydrate in eine für diese Verwendung bestimmte Form bringt.

11. Verfahren zur Herstellung von pharmazeutischen Zusammensetzungen, dadurch gekennzeichnet, daß man als Wirkstoff das (1S,2S)-N-[2,3-Dihydro-2-(1-pyrrolidinyl)-1H-inden-1-yl]-N-methyl-3-nitrobenzolacetamid und dessen Hydrochlorid oder eines seiner Hydrate oder pharmazeutisch verträglichen Additionssalze in eine für diese Verwendung bestimmte Form bringt.

12. Verfahren zur Herstellung von pharmazeutischen Zusammensetzungen, dadurch gekennzeichnet, daß man als Wirkstoff das trans-(±)-N-[5,6-Dichlor-2,3-dihydro-2-(1-pyrrolidinyl)-1H-inden-1-yl]-N-methyl-3-nitrobenzolacetamid oder eines seiner Hydrate oder seine pharmazeutisch verträglichen Salze in eine für diese Verwendung bestimmte Form bringt.
